# EUROPEAN PATENT SPECIFICATION

(11) **EP 2 029 750 B1**
(45) Date of publication and mention of the grant of the patent: **10.08.2011**
(21) Application number: 07786762.0
(22) Date of filing: 19.06.2007
(51) Int. Cl.: C12N 15/80, C12P 7/42, C12P 7/62

(54) **PRODUCTION OF PRAVASTATIN**
HERSTELLUNG VON PRAVASTATIN
PRODUCTION DE PRAVASTATINE

(30) Priority: 22.06.2006 EP 06115917
(43) Date of publication of application: 04.03.2009
(73) Proprietor: DSM IP Assets B.V., 6411 TE Heerlen (NL)
(72) Inventor: BERG, VAN DEN, Marco Alexander, 2685 EH Poeldijk (NL); MEIJRINK, Bernard, 3135 PG Vlaardingen (NL)
(74) Representative: De Vroom, Erik
(86) International application number: PCT/EP2007/056096
(87) International publication number: WO 2007/147827

(56) References cited:
- EP-A- 1 266 967
- WO-A-96/40863
- WO-A-99/10499
- US-B2- 7 056 710
- ABE Y ET AL: "Effect of increased dosage of the ML-236B (compactin) biosynthetic gene cluster on ML-236B production in Penicillium citrinum" MGG - MOLECULAR GENETICS AND GENOMICS, SPRINGER, BERLIN, DE, vol. 268, no. 1, 9 August 2002 (2002-08-09), pages 130-137, XP002400771 ISSN: 1617-4615
- WATANABE I ET AL: "Molecular approaches for production of pravastatin, a HMG-CoA reductase inhibitor: transcriptional regulation of the cytochrome p450sca gene from Streptomyces carbophilus by ML-236B sodium salt and phenobarbital" GENE: AN INTERNATIONAL JOURNAL ON GENES AND GENOMES, ELSEVIER, AMSTERDAM, NL, vol. 210, no. 1, 27 March 1998 (1998-03-27), pages 109-116, XP002290631 ISSN: 0378-1119
- ABE Y ET AL: "Functional analysis of mlcR, a regulatory gene for ML-236B (compactin) biosynthesis in Penicillium citrinum." MGG MOLECULAR GENETICS AND GENOMICS, vol. 268, no. 3, November 2002 (2002-11), pages 352-361, XP002474551 ISSN: 1617-4615
- ABE Y ET AL: "Molecular cloning and characterization of an ML-236B (compactin) biosynthetic gene cluster in Penicillium citrinum" MGG - MOLECULAR GENETICS AND GENOMICS, SPRINGER, BERLIN, DE, vol. 267, no. 5, July 2002 (2002-07), pages 636-646, XP002472854 ISSN: 1617-4615
- CHAKRAVARTI R ET AL: "Compactin - a review" APPLIED MICROBIOLOGY AND BIOTECHNOLOGY, SPRINGER VERLAG, BERLIN, DE, vol. 64, no. 5, 19 March 2004 (2004-03-19), pages 618-624, XP002400772 ISSN: 0175-7598

## Description

### Field of the Invention

The present invention relates to a one-step fermentation process for the production of pravastatin.

### Background of the invention

Cholesterol and other lipids are transported in body fluids by low-density lipoproteins (LDL) and high-density lipoproteins (HDL). Substances that effectuate mechanisms for lowering LDL-cholesterol may serve as effective antihypercholesterolemic agents because LDL levels are positively correlated with the risk of coronary artery disease. Cholesterol lowering agents of the statin class are medically very important drugs as they lower the cholesterol concentration in the blood by inhibiting HMG-CoA reductase. The latter enzyme catalyses the rate limiting step in cholesterol biosynthesis, *i.e*. the conversion of (3S)-hydroxy-3-methylglutarylco-enzyme A (HMG-CoA) to mevalonate. There are several types of statins on the market, amongst which atorvastatin, compactin, lovastatin, simvastatin and pravastatin. Whilst atorvastatin is made via chemical synthesis, the other statins mentioned above are produced either via direct fermentation or via precursor fermentation. These (precursor) fermentations are carried out by fungi of the genera *Penicillium, Aspergillus* and *Monascus.*

Pravastatin is produced via two sequential fermentations. First *Penicillium citrinum* produces compactin, of which the lacton ring is chemically hydrolyzed with sodium hydroxide; subsequently this is fed to a cultivation of *Streptomyces carbophilus,* which hydroxylates it to pravastatin. The industrial species and processes for the production of these metabolites are optimized using different methods. By this, the industrial compactin production by *Penicillium citrinum* was increased from the original 40 mg/L to 5 g/L. For the biocatalytic conversion a *Streptomyces* mutant strain with resistance to 3 g/L of mevastatin with an 80% conversion yield was obtained by Metkinen (Metkinen News March 2000, Metkinen Oy, Finland; reviewed by Manzoni and Rollini, 2002, Appl Microbiol Biotechnol 58:555-564). Although this is a viable commercial process it is far from optimal as the compactin titers are relatively low compared to, for example industrial amino acid or penicillin G titers. Also, the compactin needs to be diluted otherwise it becomes toxic for the *Streptomyces* strains used in the bioconversion (Hosobuchi et al., 1983, J Antibiotics 36:887-891) and in addition 20% of the compactin fed is not converted by the *Streptomyces* strains.

As a solution to the above problem, several publications describe a one-step fermentation process for pravastatin (WO 99/10499, US 6,274,360 and EP 1,266,967). However, as part of this invention we will demonstrate that all these suggestions to solve the problem are invalid and require an additional problem to be solved, *i.e*. the intracellular conversion of compactin to pravastatin. WO 99/10499 describes recombinant *Penicillium citrinum* strains equipped with a *Streptomyces carbophilus* gene encoding a p450 enzyme. Here it is demonstrated that strains constructed according to WO 99/10499 do not produce pravastatin, but only compactin. US 6,274,360 describes an enzyme system that can be isolated from *Actinomadura* species, which can convert compactin into pravastatin and suggest using this enzyme system in heterologous species to produce pravastatin. However, as no protein or DNA sequences are provided it is not possible to verify such claims. Moreover, such an enzyme system might consist of different polypeptides and/or needs several host specific redox-regeneration enzymes or chaperones, which will hamper purification and isolation of all right members of this enzyme system. Moreover, as discussed above, even transferring the known DNA sequence of a pravastatin catalyzing enzyme (the *Streptomyces carbophilus* p450-sca2 gene) to *Penicillium citrinum,* the compactin producer, does not result in pravastatin formation. EP 1,266,967 describes mutant strains of *Aspergillus terreus* and *Monascus ruber.* While wild-type strains produce only lovastatin, these mutants were described as producing lovastatin and/or pravastatin. As part of this invention we will demonstrate that mutant strains according to EP 1,266,967 do not produce pravastatin, but only lovastatin.

Therefore, an economically feasible way of solving the problem of a one-step fermentation process for pravastatin is not available and is extremely desirable.

The present invention provides a method for producing pravastatin comprising the steps of:
(a) transforming a *Penicillium chrysogenum* cell with a polynucleotide comprising genes mlcA, mlcB, mlcC, mlcD, mlcE, mlcf, mlcG and mlcH;
(b) transforming said *Penicillium chrysogenum* cell with a polynucleotide comprising a gene encoding compactin hydroxylase;
(c) selecting clones of transformed cells, and;
(d) cultivating said selected cells.

### Detailed description of the invention

The term "expression" includes any step involved in the production of a polypeptide and may include transcription, post-transcriptional modification, translation, post-translational modification and secretion.

The term "nucleic acid construct" is synonymous with the term "expression vector" or "cassette" when the nucleic acid construct contains all the control sequences required for expression of a coding sequence in a particular host organism.

The term "control sequences" is defined herein to include all components, which are necessary or advantageous for the expression of a polypeptide. Each control sequence may be native or foreign to the nucleic acid sequence encoding the polypeptide. Such control sequences may include, but are not limited to, a promoter, a leader, optimal translation initiation sequences (as described in Kozak, 1991, J. Biol. Chem. 266:19867-19870), a secretion signal sequence, a pro-peptide sequence, a polyadenylation sequence, a transcription terminator. At a minimum, the control sequences include a promoter, and transcriptional and translational stop signals. The control sequence may be an appropriate promoter sequence containing transcriptional control sequences. The promoter may be any nucleic acid sequence, which shows transcription regulatory activity in the cell including mutant, truncated, and hybrid promoters, and may be obtained from genes encoding extra cellular or intracellular polypeptides. The promoter may be either homologous or heterologous to the cell or to the polypeptide. The promoter maybe derived from the donor species or from any other source. An alternative way to control expression levels in eukaryotes is the use of introns. Higher eukaryotes have genes consisting of exons and introns. The term "exons" is defined herein to include all components of the Open Reading Frame (ORF), which are translated into the protein. The term "introns" is defined herein to include all components, which are not comprised within the Open Reading Frame. The term "Open Reading Frame" is defined herein as a polynucleotide starting with the sequence ATG, the codon for methionine, followed by a consecutive series of codons encoding all possible amino acids and after a certain number interrupted by a termination codon. This Open Reading Frame can be translated into a protein. A polynucleotide containing a gene isolated from the genome is a so-called genomic DNA or gDNA sequence of that gene, including all exons and introns. A polynucleotide containing a gene isolated from mRNA via reverse transcriptase reactions is a so-called copy DNA or cDNA sequence of that gene, including only the exons, while the introns are spliced out through the cells machinery. This latter type of DNA is of particular use when expressing eukaryotic genes of interest in prokaryotic hosts. Variants of both types of DNA can also be made synthetically, which opens the possibility to either alter the exact nucleotide sequence of the introns or vary the number of introns in the gene of interest. This also opens the possibility of adding introns to genes of interest from prokaryotic origin to facilitate or improve expression in eukaryotic hosts. Also, introns can be introduced in the above named control sequences, like a promoter, a polyadenylation site or a transcription terminator. The presence, absence, variation or introduction of introns is a means of regulating gene expression levels in eukaryotes.

The term "operably linked" is defined herein as a configuration in which a control sequence is appropriately placed at a position relative to the coding sequence of the DNA sequence such that the control sequence directs the production of a polypeptide.

The term "pravastatin" is defined herein as the 6'-hydroxyl variant of compactin with an α- or β-configuration, or a mixture of both α- and β-configurations and includes both the closed structure (with a lactone ring) and the open structure (with a hydroxycarboxylic acid moiety).

It is an object of the present invention to provide a method for a one-step fermentation process of pravastatin.

For the method of the present invention there is provided a pravastatin producing strain having at least one of the following characteristics:
- containing all genes necessary for compactin biosynthesis,
- containing all genes necessary for compactin into pravastatin conversion (*i.e*. a compactin hydroxylase expressing host cell),
- capable of producing pravastatin in one fermentation

In the context of this invention "pravastatin producing cells" are defined by cells that display at least one of the above characteristics, preferable two of the above characteristics and most preferable all of the above characteristics.

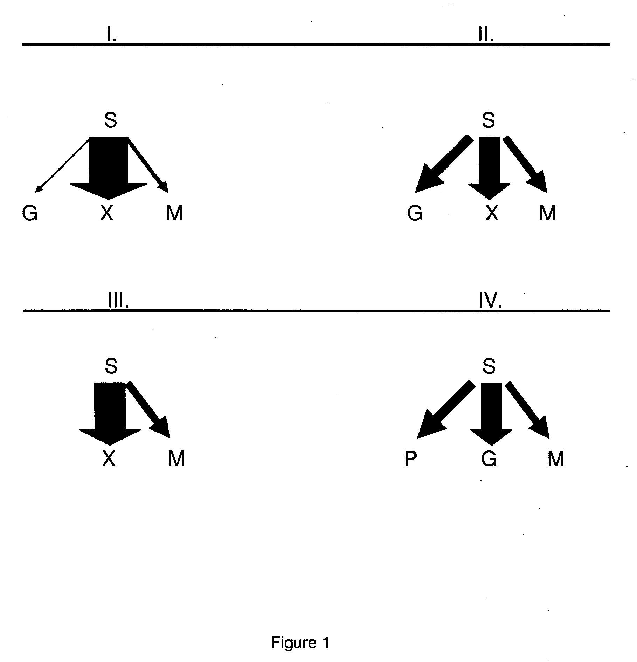

In one embodiment there is provided a compactin producing host cell derived from a production strain, *Penicillium chrysogenum.* This organism underwent several rounds of classical strain improvement and subsequent process adaptations and improvements to come to the current high titer penicillin G fermentation processes. The numerous changes in the DNA of the organism resulted not only in an increased flux and yield towards the product penicillin G (see Figure 1), but moreover also resulted in morphological changes and adaptations to the harsh conditions in 150,000-liter fermentation vessels (*i.e*. oxygen limitation, shear forces, glucose limitation and the like). By deleting the β-lactam biosynthetic machinery, a strain is obtained that is devoid of any β-lactam production capability, but still retains all the mutations that result in the good performance on industrial scale, such as resistance to shear forces, suitability for scaling up, high metabolic flux towards metabolites, adapted to a defined medium and to industrial down stream processing, and low viscosity profile (*i.e*. morphological, regulatory and metabolic mutations). In the *Penicillin chrysogenum* strain of the present invention, at least the β-lactam biosynthetic genes pcbC, encoding for isopenicillin N synthase, are inactivated. Preferably, also the other β-lactam biosynthetic genes, *pcbAB*, encoding for L-(α-aminoadipyl)-L-cysteinyl-D-valine synthetase, and/or *penDE,* encoding for acyl-coenzyme A:isopenicillin N acyltransferase, are inactivated. More preferably, the genes are inactivated by removal of part of the genes. Most preferred is that the gene sequences are completely removed. As complete removal of these genes leads to *Penicillium chrysogenum* strains that are devoid of any β-lactam biosynthetic capacity and therefore are very useful strains for producing all sorts of products. Despite the fact that industrial organisms can be very cumbersome to work with, this *Penicillium chrysogenum* strain is surprisingly well transformable and capable of producing statins at titers much higher than the natural producing host cells.

Although not mandatory for the present invention, preferably the *Penicillium chrysogenum* mutant is obtained from an organism capable of producing in an industrial environment. Such organisms typically can be defined as having high productivities and/or high yield of product on amount of carbon source consumed and/or high yield of product on amount of biomass produced and/or high rates of productivity and/or high product titers. Such organisms are extremely useful for conversion into a host cell for compactin. For penicillin G producing *Penicillium chrysogenum* strains for instance, such high titers are titers higher than 1.5 g/L penicillin G, preferably higher than 2 g/L penicillin G, more preferably higher than 3 g/L penicillin G, most preferably higher than 4 g/L penicillin G. The aforementioned values apply to fermentation titers after 96 h in complex fermentation medium (contains per liter: lactose, 40 g/L; corn steep solids, 20 g/L; CaCO₃, 10 g/L; KH₂PO₄, 7 g/L; phenylacetic acid, 0.5 g/L; pH 6.0). Suitable industrial strains are strains as mentioned in the experimental part (General Methods).

All industrial strain lineages of *Penicillium chrysogenum* underwent numerous rounds of classical strain improvement resulting in three general types of mutations:
(i) Direct amplification of the biosynthetic genes resulting in increased activity of the enzymes of the penicillin metabolite pathway
(ii) Modifications in primary metabolism genes, ultimately resulting in various adapted metabolic rearrangements, all leading to higher a higher flux towards the end product. Examples: increased synthesis of amino acid building blocks, decreased consumption of phenylacetic acid and the like.
(iii) Cell structure modifications, resulting in alteration of morphology, membrane composition, organelles organization and thereby 'facilitating' high metabolic fluxes and fermentation at industrial scale. Examples: increased numbers of peroxisomes, which are one of the 'assembly lines' of penicillin synthesis.

There is a significant distinction on DNA level in the type of mutations of class (i) as compared to classes (ii) and (iii). While the latter two classes are mostly isolated mutations, deletions, duplications and/or alterations on base pair level, the mutation in class (i) is a very distinct amplification of a 60 to 100 kb region, resulting in several direct and inverted repeats on the genome. This sometimes causes a significant genetic instability, resulting in an instable and changing population. In fact this means that in a given penicillin production strain all mutations of class (ii) and (iii) are fixed, but the exact copy number of the mutation of class (i) can fluctuate. Using this principle and techniques known to the ones trained in the art, stable isolates can be obtained where only one copy of the penicillin biosynthetic genes is still present. Depending on the copy number of the starting strain this situation can be obtained in one, two, three or several rounds of screening and selection. For this specific characteristic the isolate is then comparable to the type strain of the species, NRRL1951, and its first descendants after classical strain improvement, up to Wisconsin 54-1255, all of which contain one copy of the penicillin biosynthetic genes. The major difference is that the one-copy isolate derived from the high producing strain still contains all the other mutations of class (ii) and (iii) making it an industrial high producing strain as compared to the strains from NRRL1951 to Wisconsin 54-1255. Subsequently, the last set of penicillin biosynthetic genes can be deleted using state-of-the-art recombination techniques. A detailed overview of these steps is given in the examples and summarized in the following steps:
(a) Isolating an isolate with a single genomic copy of the penicillin gene cluster from a *Penicillium* strain
(b) Deleting gene *pcbC* from the isolate obtained in step (a)
(c) Optionally deleting genes *pcbAB* and/or *penDE* from the isolate obtained in steps (a) or (b)

The genes can be partly inactivated. More preferably, the gene sequences are completely removed. As complete removal of these genes leads to *Penicillium chrysogenum* strains that are devoid of any β-lactam biosynthetic capacity and therefore are very useful strains for producing all sorts of products. Recombination techniques that can be applied are well known for the ones trained in the art (*i.e*. Single Cross Over or Double Homologous Recombination).

A preferred strategy for deletion and replacement is the gene replacement technique described in EP 357,127. The specific deletion of a gene and/or promoter sequence is preferably performed using the *amdS* gene as selection marker gene as described in EP 635,574. By means of counter selection on fluoroacetamide media (EP 635,574), the resulting strain is selection marker free and can be used for further gene modifications. Alternatively or in combination with other mentioned techniques, a technique based on *in vivo* recombination of cosmids in *Escherichia coli* can be used, as by Chaveroche et al. (2000, Nucl Acids Res, 28, E97). This technique is applicable to other filamentous fungi like for example *Penicillium chrysogenum.* Also, the same principle for removing amplified genome fragments can be applied to other industrial production species in which classical strain improvement programs have induced gene and genome duplications. Also, here additional mutations of class (ii) and (iii) are fixed and make sure that the strains can thrive in industrial fermentation processes.

Such *Penicillium chrysogenum* cells can be equipped with the genes encoding all proteins and enzymes necessary for compactin biosynthesis. Nine genes of *Penicillium citrinum* are described as being involved in compactin biosynthesis (Abe et al., 2002, Mol Genet Genomics 267:636-646; Abe et al., 2002, Mol Genet Genomics 268:130-137): *micA,* encoding a polyketide synthase; *mlcB,* encoding a polyketide synthase; *mlcC,* encoding P450 monooxygenase; *mlcD,* encoding a HMG-CoA reductase; *mlcE,* encoding an efflux pump; *mlcF,* encoding an oxidoreductase; *mlcG,* encoding a dehydrogenase; *mlcH,* encoding transesterase; *mlcR,* encoding a transcription factor.

To obtain a compactin producing host cell, the isolated *Penicillium chrysogenum* strains are transformed with at least one of the above genes, preferable with two or more of the above genes, more preferable with all of the above genes.

In another embodiment the same principle can be applied to come to a suitable compactin producing host cell of other eukaryotic species, and their industrial derivatives, like, but not limited to: *Aspergillus niger, Aspergillus terreus, Penicillium brevicompactum, Penicillium citrinum, Aspergillus oryzae, Trichoderma reesei, Chrysosporium lucknowense, Saccharomyces cerevisiae, Kluyveromyces lactis, Monascus ruber,* Monascus *paxii, Mucor hiemalis, Pichia ciferrii* and *Pichia pastoris.* The industrial derivatives of these species underwent various rounds of classical mutagenesis, followed by screening and selection for improved industrial production characteristics, which make them of particular use for the present invention. By removing (*i.e*. deleting) parts of or complete pathways of unwanted products the strains remain their desired industrial fermentation characteristics and high flux to metabolites (including enzymes).

In yet another embodiment the production of compactin can be improved by using homologous proteins with improved kinetic features. Such a "homologue" or "homologous sequence" is defined as a DNA sequence encoding a polypeptide that displays at least one activity of the polypeptide encoded by the original DNA sequence isolated from the donor species and has an amino acid sequence possessing a degree of identity to the amino acid sequence of the protein encoded by the specified DNA sequence. A polypeptide having an amino acid sequence that is "substantially homologous" to the compactin biosynthetic genes are defined as polypeptides having an amino acid sequence possessing a degree of identity to the specified amino acid sequence of at least 25%, more preferably at least 30%, more preferably at least 40%, more preferably at least 50%, still more preferably at least 60%, still more preferably at least 70%, still more preferably at least 80%, still more preferably at least 90%, still more preferably at least 98% and most preferably at least 99%, the substantially homologous peptide displaying activity towards the synthesis of compactin and/or compactin-precursors. Using this approach various advantages are obtained such as to overcome feedback inhibition, improvement of secretion and reduction of byproduct formation. A homologous sequence may encompass polymorphisms that may exist in cells from different populations or within a population due to natural allelic or intra-strain variation. A homologue may further be derived from a species other than the species where the specified DNA sequence originates from, or may be artificially designed and synthesized. DNA sequences related to the specified DNA sequences and obtained by degeneration of the genetic code are also part of the invention. Homologues may also encompass biologically active fragments of the full-length sequence.

Of particular interest are homologous sequences isolated by synthetic means. By this method all possible variants of the genes to be introduced in the compactin producing host cell can be designed in silico. This opens the opportunity to adapt the codon usage of the genes such that they are optimally expressed in the compactin producing host cell; remove and introduce relevant sequences for restriction enzymes and/or site-specific recombinases; make different combinations of the genes, and the like.

For the purpose of the present invention, the degree of identity between two amino acid sequences refers to the percentage of amino acids that are identical between the two sequences. The degree of identity is determined using the BLAST algorithm, which is described in Altschul, et al., J. Mol. Biol. 215: 403-410 (1990). Software for performing BLAST analyses is publicly available through the National Center for Biotechnology Information (http://www.ncbi.nlm.nih.gov/). The BLAST algorithm parameters W, T, and X determine the sensitivity and speed of the alignment. The BLAST program uses as defaults a word length (W) of 11, the BLOSUM62 scoring matrix (see Henikoff & Henikoff, Proc. Natl. Acad. Sci. USA 89: 10915 (1989)) alignments (B) of 50, expectation (E) of 10, M=5, N=-4, and a comparison of both strands.

Substantially homologous polypeptides may contain only conservative substitutions of one or more amino acids of the specified amino acid sequences or substitutions, insertions or deletions of non-essential amino acids. Accordingly, a non-essential amino acid is a residue that can be altered in one of these sequences without substantially altering the biological function. For example, guidance concerning how to make phenotypically silent amino acid substitutions is provided in Bowie, J.U. et al., Science 247:1306-1310 (1990) wherein the authors indicate that there are two main approaches for studying the tolerance of an amino acid sequence to change. The first method relies on the process of evolution, in which mutations are either accepted or rejected by natural selection. The second approach uses genetic engineering to introduce amino acid changes at specific positions of a cloned gene and selects or screens to identify sequences that maintain functionality. As the authors state, these studies have revealed that proteins are surprisingly tolerant of amino acid substitutions. The authors further indicate which changes are likely to be permissive at a certain position of the protein. For example, most buried amino acid residues require non-polar side chains, whereas few features of surface side chains are generally conserved. Other such phenotypically silent substitutions are described in Bowie et al, and the references cited therein.

The term "conservative substitution" is intended to mean that a substitution in which the amino acid residue is replaced with an amino acid residue having a similar side chain. These families are known in the art and include amino acids with basic side chains (e.g. lysine, arginine and histidine), acidic side chains (e.g. aspartic acid, glutamic acid), uncharged polar side chains (e.g., glycine, asparagines, glutamine, serine, threonine, tyrosine, cysteine), non-polar side chains (e.g., alanine, valine, leucine, isoleucine, proline, phenylalanine, methionine, tryptophan), β-branched side chains (e.g., threonine, valine, isoleucine) and aromatic side chains (e.g., tyrosine, phenylalanine tryptophan, histidine).

Additionally, biosynthetic gene clusters that are not homologous, but follow the same biosynthetic building principle for statin synthesis can be used.

The nucleic acid constructs of the present invention, *e.g*. expression constructs, contain at least one gene of interest, but in general contain several genes of interest; each operably linked to one or more control sequences, which direct the expression of the encoded polypeptide in the compactin producing host cell. The nucleic acid constructs may be on one DNA fragment or on separate fragments. To obtain the highest possible productivity a balanced expression of all genes of interests is crucial. Therefore, a range of promoters can be useful. Preferred promoters for application filamentous fungal cells like *Penicillium chrysogenum* are known in the art and can be, for example, the promoters of the gene(s) derived *Penicillium citrinum;* the glucose-6-phosphate dehyrogenase gpdA promoters; the *Penicillium chrysogenum pcbAB, pcbC* and *penDE* promoters; protease promoters such as *pepA, pepB, pepC*; the glucoamylase *glaA* promoters; amylase *amyA, amyB* promoters; the catalase *cstR* or *catA* promoters; the glucose oxidase *goxC* promoter; the beta-galactosidase *lacA* promoter, the α-glucosidase *aglA* promoter; the translation elongation factor *tefA* promoter, xylanase promoters such as *xlnA, xlnB, xlnC, xlnD;* cellulase promoters such as *eglA, eglB, cbhA;* promoters of transcriptional regulators such as *areA, creA, xlnR, pacC, prtT, alcR,* or any other. Said promoters can easily be found by the skilled person, amongst others, at the NCBI Internet website (http://www.ncbi.nlm.nih.gov/entrez/). In case of compactin producing host cells derived from other than filamentous fungal species the choice of promoters will be determined by the choice of the host.

In a preferred embodiment, the promoters may be derived from genes, which are highly expressed (defined herein as the mRNA concentration with at least 0.5% (w/w) of the total cellular mRNA). The promoters may be derived from genes, which are medium expressed (defined herein as the mRNA concentration with at least 0.01% until 0.5% (w/w) of the total cellular mRNA). In another preferred embodiment, the promoters may be derived from genes, which are low expressed (defined herein as the mRNA concentration lower than 0.01% (w/w) of the total cellular mRNA).

In a still more preferred embodiment micro array data is used to select genes, and thus promoters of those genes, that have a certain transcriptional level and regulation. In this way one can adapt the gene expression cassettes optimally to the conditions it should function in. These promoter fragments can be derived from many sources, *i.e*. different species, PCR amplified, synthetically and the like.

In the most preferred embodiment, for optimal production in all compactin producing host cells, the original *Penicillium citrinum* promoters are replaced by promoters which are not under control by the pathway specific transcription regulator, encoded by the gene *mlcR.* By this the gene *mlcR* is not necessary to include in a compactin producing host cell and an alternative control mechanism can be introduced.

The control sequence may also include a suitable transcription termination sequence, a sequence recognized by a eukaryotic cell to terminate transcription. The terminator sequence is operably linked to the 3'-terminus of the nucleic acid sequence encoding the polypeptide. Any terminator, which is functional in the cell, may be used in the present invention. Preferred terminators for filamentous fungal cells are obtained from the genes encoding *Aspergillus* oryzae TAKA amylase; the *Penicillium chrysogenum pcbAB, pcbC* and *penDE* terminators; *Aspergillus niger* glucoamylase; *Aspergillus nidulans* anthranilate synthase; *Aspergillus niger* alpha-glucosidase; *Aspergillus nidulans trpC* gene; *Aspergillus nidulans amdS; Aspergillus nidulans gpdA; Fusarium oxysporum* trypsin-like protease. Even more preferred terminators are the ones of the gene(s) derived from the natural producer, *Penicillium citrinum.* In case of compactin producing host cells derived from other than filamentous fungal species the choice of termination sequences will be determined by the choice of the host.

The control sequence may also be a suitable leader sequence, a non-translated region of an mRNA that is important for translation by the cell. The leader sequence is operably linked to the 5'-terminus of the nucleic acid sequence encoding the polypeptide. Any leader sequence, which is functional in the cell, may be used in the present invention. Preferred leaders for filamentous fungal cells are obtained from the genes encoding *Aspergillus oryzae* TAKA amylase and *Aspergillus nidulans* triose phosphate isomerase and *Aspergillus niger* glaA.

The control sequence may also be a polyadenylation sequence, which is operably linked to the 3'-terminus of the nucleic acid sequence and which, when transcribed, is recognized by the filamentous fungal cell as signal to add polyadenosine residues to transcribed mRNA. Any polyadenylation sequence, which is functional in the cell, may be used in the present invention. Preferred polyadenylation sequences for filamentous fungal cells are obtained from the genes encoding *Aspergillus* oryzae TAKA amylase, *Aspergillus niger* glucoamylase, *Aspergillus nidulans* anthranilate synthase, *Fusarium oxysporum* trypsin-like protease and *Aspergillus niger* alpha-glucosidase.

For a polypeptide to be secreted, the control sequence may also include a signal peptide-encoding region, coding for an amino acid sequence linked to the amino terminus of the polypeptide, which can direct the encoded polypeptide into the cell's secretory pathway. The 5'-end of the nucleic acid coding sequence may inherently contain a signal peptide-coding region naturally linked in translation reading frame with the segment of the coding region, which encodes the secreted polypeptide. Alternatively, the 5'-end of the coding sequence may contain a signal peptide-coding region, which is foreign to the coding sequence. The foreign signal peptide-coding region may be required where the coding sequence does not normally contain a signal peptide-coding region. Alternatively, the foreign signal peptide-coding region may simply replace the natural signal peptide-coding region in order to obtain enhanced secretion of the polypeptide.

In case of eukaryotic compactin producing host cells the control sequence may include organelle targeting signals. Such a sequence is encoded by an amino acid sequence linked to the polypeptide, which can direct the final destination (*i.e.* compartment or organelle) within the cell. The 5'- or 3'-end of the coding sequence of the nucleic acid sequence may inherently contain these targeting signals coding region naturally linked in translation reading frame with the segment of the coding region, which encodes the polypeptide. The various sequences are well known to the persons trained in the art and can be used to target proteins to compartments like mitochondria, peroxisomes, endoplasmatic reticulum, golgi apparatus, vacuole, nucleus and the like. The nucleic acid construct may be an expression vector. The expression vector may be any vector (*e.g*. a plasmid or virus), which can be conveniently subjected to recombinant DNA procedures and can bring about the expression of the nucleic acid sequence encoding the polypeptide. The choice of the vector will typically depend on the compatibility of the vector with the cell into which the vector is to be introduced. The vectors may be linear or closed circular plasmids. The vector may be an autonomously replicating vector, *i.e*. a vector, which exists as an extra chromosomal entity, the replication of which is independent of chromosomal replication, *e.g*. a plasmid, an extra chromosomal element, a mini chromosome, or an artificial chromosome. An autonomously maintained cloning vector for a filamentous fungus may comprise the AMA1-sequence (see *e.g*. Aleksenko and Clutterbuck (1997), Fungal Genet. Biol. 21: 373-397). Alternatively, the vector may be one which, when introduced into the cell, is integrated into the genome and replicated together with the chromosome(s) into which it has been integrated. The integrative cloning vector may integrate at random or at a predetermined target locus in the chromosomes of the host cell. In a preferred embodiment of the invention, the integrative cloning vector comprises a DNA fragment, which is homologous to a DNA sequence in a predetermined target locus in the genome of host cell for targeting the integration of the cloning vector to this predetermined locus. In order to promote targeted integration, the cloning vector is preferably linearized prior to transformation of the host cell. Linearization is preferably performed such that at least one but preferably either end of the cloning vector is flanked by sequences homologous to the target locus. The length of the homologous sequences flanking the target locus is preferably at least at least 0.1 kb, even preferably at least 0.2 kb, more preferably at least 0.5 kb, even more preferably at least 1 kb, most preferably at least 2 kb.

The efficiency of targeted integration of a nucleic acid construct into the genome of the host cell by homologous recombination, *i.e*. integration in a predetermined target locus, is preferably increased by augmented homologous recombination abilities of the host cell. Such phenotype of the cell preferably involves a deficient *hdfA* or *hdfB* gene as described in WO 05/95624. WO 05/95624 discloses a preferred method to obtain a filamentous fungal cell comprising increased efficiency of targeted integration.

The vector system may be a single vector or plasmid or two or more vectors or plasmids, which together contain the total DNA to be introduced into the genome of the host cell. However in the present invention the constructs are preferably integrated in the genome of the host strain. As this is a random process this even can result in integration in genomic loci, which are highly suitable to drive gene expression, resulting in high amounts of enzyme and subsequently in high productivity.

Fungal cells may be transformed by protoplast formation, protoplast transformation, and regeneration of the cell wall. Suitable procedures for transformation of fungal host cells are described in EP 238,023 and Yelton et al. (1984, Proc. Natl. Acad. Sci. USA 81:1470-1474). Suitable procedures for transformation of filamentous fungal host cells using *Agrobacterium tumefaciens* are described by de Groot M.J. et al. (1998, Nat Biotechnol 16:839-842; Erratum in: 1998, Nat Biotechnol 16:1074). Other methods like electroporation, described for *Neurospora crassa,* may also be applied.

Fungal cells are transformed using co-transformation, *i.e*. along with gene(s) of interest also a selectable marker gene is transformed. This can be either physically linked to the gene of interest (*i.e*. on a plasmid) or on a separate fragment. Following transfection transformants are screened for the presence of this selection marker gene and subsequently analyzed for the presence of the gene(s) of interest. A selectable marker is a product, which provides resistance against a biocide or virus, resistance to heavy metals, prototrophy to auxotrophs and the like. Useful selectable markers include *amdS* (acetamidase), *argB* (ornithinecarbamoyltransferase), *bar* (phosphinothricinacetyltransferase), *hygB* (hygromycin phosphotransferase), *niaD* (nitrate reductase), *pyrG* (orotidine-5'-phosphate decarboxylase), *sC* or *sutB* (sulfate adenyltransferase), *trpC* (anthranilate synthase), ble (phleomycin resistance protein), or equivalents thereof.

In another embodiment the same principle can be applied to come to a suitable compactin producing host cell of prokaryotic species, and their industrial derivatives, like, but not limited to: *Streptomyces clavuligerus, Streptomyces avermitilis, Streptomyces peucetius, Streptomyces coelicolor, Streptomyces lividans, Streptomyces carbophilus, Amycolatopis orientalis, Corynebacterium glutamicum* and *Escherichia coli.* The industrial derivatives of these species underwent various rounds of classical mutagenesis, followed by screening and selection for improved industrial production characteristics, which make them of particular use for the present invention. By removing (i.e. deleting) parts of or complete pathways of unwanted products the strains remain their desired industrial fermentation characteristics and high flux to metabolites (including enzymes). Here, all the genes selected from the list of *Penicillium citrinum* compactin genes (*mlcA, mlcB, mlcC, mlcD, mlcE, mlcF, mlcG, mlcH* and/or *mlcR*) need be modified by state-of-the art methods to be functionally expressed in prokaryotic cells. The ones trained in the art will understand that this involves various steps comparable as outlined above for eukaryotes, including, but not limited to:
- obtaining cDNA or synthetic DNA (to exclude eukaryotic introns)
- optionally using codon-optimization
- equipping with promoters functional in prokaryotes
- equipping with terminators functional in prokaryotes
- introducing via vectors functional in prokaryotes

In another embodiment of the invention there is provided a host microorganism comprising the genes necessary for converting compactin into pravastatin. The penicillin producing parent strains of the *Penicillium chrysogenum* compactin producing host cell of example 3 are surprisingly particular useful for converting compactin onto pravastatin. The *Streptomyces carbophilus* gene P-450sca-2, encoding a p450 enzyme, was described by Watanabe et al. (1995, Gene 163:81-85) as being the catalyst for the compactin to pravastatin conversion. So far this gene was only successfully expressed in *Streptomyces carbophilus* and *Streptomyces lividans* (Watanabe at al., 1995). p450 enzymes are known to need a rather specific co-factor regeneration system (see for review Pylypenko and Schlichting, 2004, Annu. Rev. Biochem. 73:991-1018), so for a good heterologous expression this regeneration system needs to be co-introduced (see for example Kubota et al., 2005, Biosci. Biotechnol. Biochem. 69:2421-2430). Surprisingly, compactin producing *Penicillium chrysogenum* host cells equipped with only the structural P-450sca-2 gene from *Streptomyces carbophilus* are capable of converting compactin into pravastatin and therefore the first example of a one-step fermentation of pravastatin. The production level of pravastatin can be significantly improved by also equipping said *Penicillium chrysogenum* host cells with a ferredoxin gene. Preferably, said ferredoxin gene is from *Streptomyces helvaticus* as described in US20060172383 or from *Streptomyces carbophilus.* The scope of the invention is not limited to these specific examples.

The present invention provides a method for producing a compound of interest comprising the steps of:
(a) Transforming a host cell with a polynucleotide comprising the genes of interest encoding the minimal requirements for compactin biosynthesis;
(b) Transforming said host cell with a polynucleotide comprising the gene of interest encoding compactin hydroxylase and/or with a polynucleotide comprising a DNA sequence affecting the expression of the gene of interest;
(c) Selecting clones of transformed cells;
(d) Cultivating said selected cells;
(e) Feeding nutrient sources to said cultivated cells, and;
(f) Isolating the compound of interest from said cultivations.

In a preferred embodiment, the compound of interest is pravastatin.

In another embodiment the production of pravastatin in a compactin hydroxylase expressing host cell can be improved by using homologous proteins with improved kinetic features. Such a "homologue" or "homologous sequence" is defined as a DNA sequence encoding a polypeptide that displays at least one activity of the polypeptide encoded by the original DNA sequence isolated from the donor species and has an amino acid sequence possessing a degree of identity to the amino acid sequence of the protein encoded by the specified DNA sequence. A polypeptide having an amino acid sequence that is "substantially homologous" to the compactin hydroxylase genes are defined as polypeptides having an amino acid sequence possessing a degree of identity to the specified amino acid sequence of at least 25%, more preferably at least 30%, more preferably at least 40%, more preferably at least 50%, still more preferably at least 60%, still more preferably at least 70%, still more preferably at least 80%, still more preferably at least 90%, still more preferably at least 98% and most preferably at least 99%, the substantially homologous peptide displaying activity towards the synthesis of pravastatin. Using this approach various advantages are obtained such as to overcome feedback inhibition, improvement of secretion and reduction of byproduct formation. A homologous sequence may encompass polymorphisms that may exist in cells from different populations or within a population due to natural allelic or intra-strain variation. A homologue may further be derived from a species other than the species where the specified DNA sequence originates from, or may be artificially designed and synthesized. DNA sequences related to the specified DNA sequences and obtained by degeneration of the genetic code are also part of the invention. Particularly important are homologous sequences isolated synthetically. By this method all possible variants of the genes encoding suitable p450 enzymes can be designed in silico. This opens the opportunity to adapt the codon usage of the genes in such a way that they are optimally expressed in either the compactin producing host cell and/or the compactin hydroxylase expressing host cell; remove and introduce relevant sequences for restriction enzymes and/or site-specific recombinases; make different combinations of the genes; etc. Alternatively, one can use in vitro evolutionary approaches like error prone PCR, family shuffling and/or directed evolution as methods to obtain homologous sequences with improved kinetic properties. Homologues may also encompass biologically active fragments of the full-length sequence. Additionally, genes that are not homologous, but do catalyze the formation from pravastatin from compactin or any of the compactin-precursors can be used.

In a further embodiment the efficiency of the compactin to pravastatin conversion may be improved by isolating a specific redox regenerating system, needed for the p450 enzyme, and introducing this in the compactin hydroxylase expressing host cell. The methods of introducing such a system in the host cell are the same as described for introducing the compactin hydroxylase and outlined above. Such redox regenerating system may be obtained from the same species as from which the polynucleotide encoding the compactin hydroxylase (*i.e*. p450) may be obtained or heterologously expressed in; examples of which are *Penicillium* species (*i.e. Penicillium chrysogenum, Penicillium citrinum*), *Aspergillus* species (*i.e. Aspergillus niger, Aspergillus nidulans, Aspergillus terreus*), *Mucor* species (*i.e. Mucor hiemalis*), *Monascus* species (*i.e. Monascus ruber, Monascus paxii*), *Streptomyces* species (*i.e*. *Streptomyces carbophilus, Streptomyces flavidovirens, Streptomyces coelicolor, Streptomyces lividans, Streptomyces exfoliates, Streptomyces avermitilis, Streptomyces clavuligerus*), *Amycolatopsis* species (*i.e. Amycolatopsis orientalis* NRRL 18098, *Amycolatopsis orientalis* ATCC 19795), *Bacillus* species (*i.e. Bacillus subtilus, Bacillus amyloliquefaciens, Bacillus licheniformis*), *Corynebacterium* species (*i.e. Corynebacterium glutamicum*), or *Escherichia* species (*i.e. Escherichia coli*). Also, alternative systems can be applied. Examples of alternative systems are, but not limited to, integrating the compactin hydroxylase of the present invention in a class IV p450 system, thereby fusing it to the redox partners (see for example Roberts et al., 2002, J Bacteriol 184:3898-3908 and Nodate et al., 2005, Appl Microbiol Biotechnol Sep 30;:1-8 [Epub ahead of print]) or by NAD(P)H generating non-p450 linked enzymes like phosphite dehydrogenase (Johannes et al., 2005, Appl Environ Microbiol. 71:5728-5734.) or by non-enzymatic means (see for example Hollmann et al., 2006, Trends Biotechnol 24:163-171). The host cell thus obtained may be used for producing pravastatin.

### Legends to the Figures

Figure 1 is a schematic representation of the invention. Wild type (WT) strains have a fixed ratio to split the incoming carbon over growth, product (penicillin G) and maintenance. In industrial strains this balance is shifted towards product. In the compactin producing host cells the penicillin G pathway is removed, so the carbon flux is rebalanced between growth and maintenance. In the new product strain, the industrial carbon flux balance is restored by introducing a new product pathway. Legend: I = wild type *Penicillium chrysogenum* strain; II = Industrial *Penicillium chrysogenum* strain; III = *Penicillium chrysogenum* β-lactam free strain; IV = *Penicillium chrysogenum* β-lactam free strain producing a new product; S = carbon source (*i.e.* sugar); G = penicillin G; X = biomass; M = Maintenance; P = Compactin and/or Pravastatin.

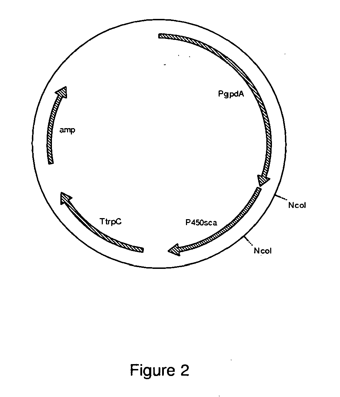

Figure 2 shows the p-450sca-2 expression vector pANp450a used to express the *Streptomyces carbophilus* p-450sca-2 enzyme in *Penicil*/*ium chrysogenum* and *Penicillium citrinum.*

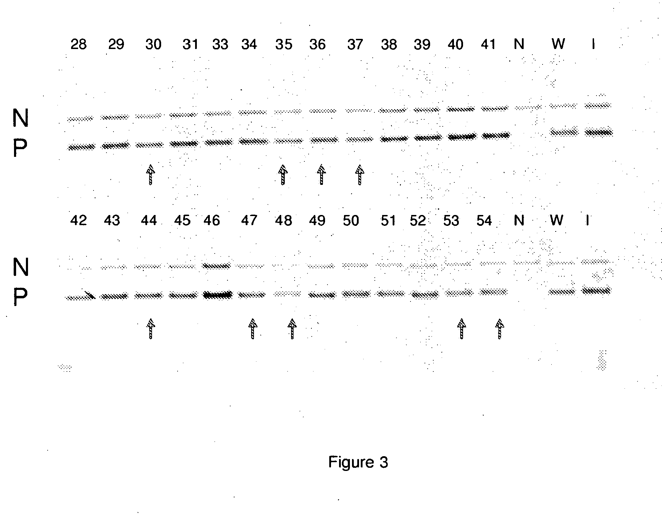

Figure 3 shows a Southern blot analysis of industrial *Penicillium chrysogenum* isolates with a single copy of the penicillin biosynthetic gene cluster. Legend: # = isolate number, N = npe10; W = Wis54-1255; I = intermediate parent; N = *niaA* gene fragment; P = *pcbC* gene fragment.

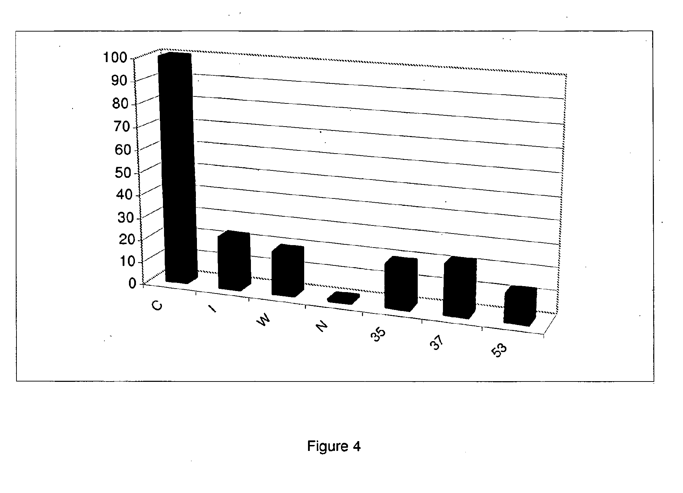

Figure 4 shows the relative penicillin V titers of various strains grown in shake flasks on mineral media with phenoxyacetic acid. On the Y-asix the percentage of penicillin V is given (level of the industrial parent set at 100). Legend: C = industrial parent; I = intermediate parent; W = Wis54-1255; N = npe10: #= isolate number.

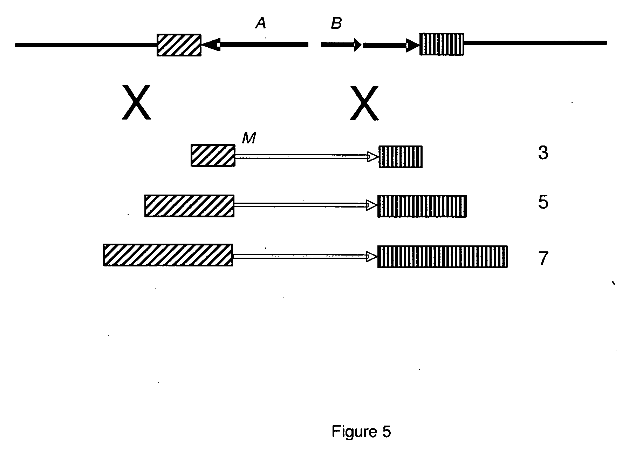

Figure 5 is a representation of the deletion strategy to remove the last copy of the penicillin gene cluster from derivatives of industrial *Penicillium chrysogenum* strains. Legend: A = pcbAB gene; B = pcbC gene; C = penDE gene; M = amdS gene cassette; 3 = 3 kb flank length; 5 = 5 kb flank length; 7 = 7 kb flank length. The hatched areas indicate the homologous flanking regions; diagonal hatches indicate the left flanking and standing hatches indicate the right flanking.

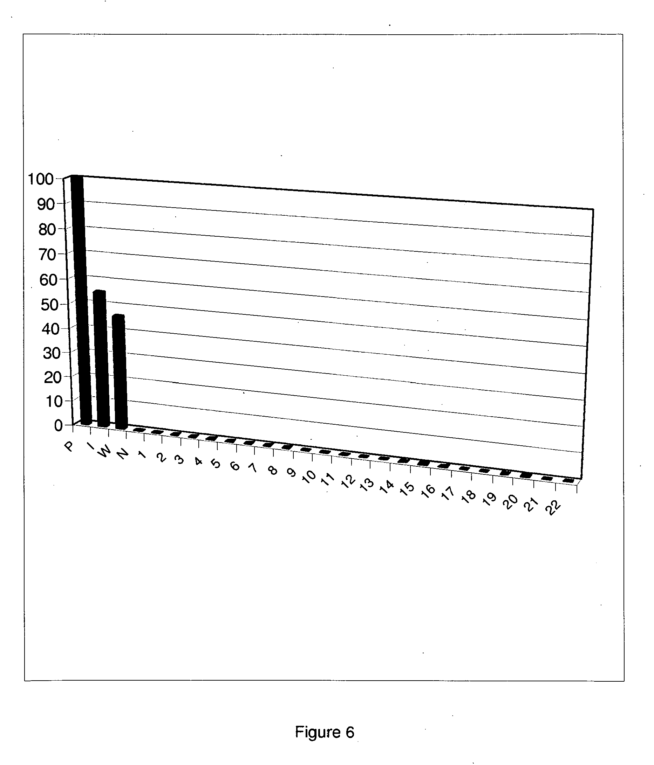

Figure 6 shows relative penicillin G titers of various strains grown in shake flasks on mineral media with phenylacetic acid. On the Y-axis the percentage of penicillin G is given with the level of the industrial parent set at 100. Legend: C = industrial parent; I = intermediate parent; W = Wis54-1255; N = npe10; # = isolate number.

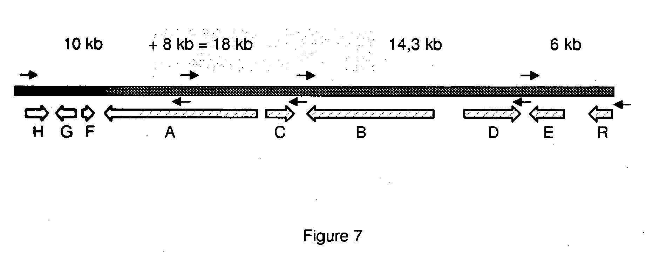

Figure 7 is a schematic representation of the compactin gene cluster (length is 38231 bp). The dashed arrows indicate the genes and there orientation. The small solid arrows indicate the position of the PCR primers used in the cloning strategy. The sizes on top indicate the length of the fragments amplified via PCR (the 10 and 8 kb fragment are combined via several cloning steps to one 18 kb fragment, see examples for details). Legend: A *= mlcA* gene; B = *mlcB* gene; C = *mlcC* gene; D = *mlcD* gene; E = *mlcE* gene; F = *mlcf* gene; G = *mlcG* gene; H= *mlcH* gene; R = *mlcR* gene.

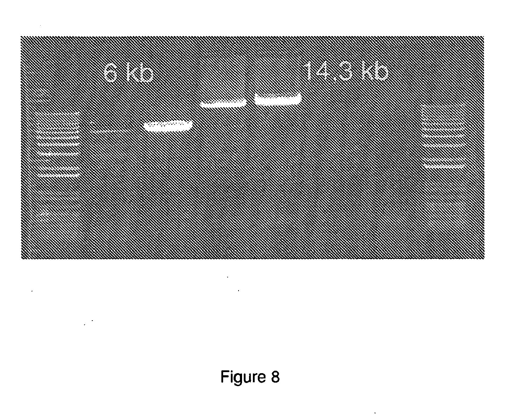

Figure 8 shows the PCR amplification of the middle part (14.3 kb) and right part (6 kb) of the compactin gene cluster.

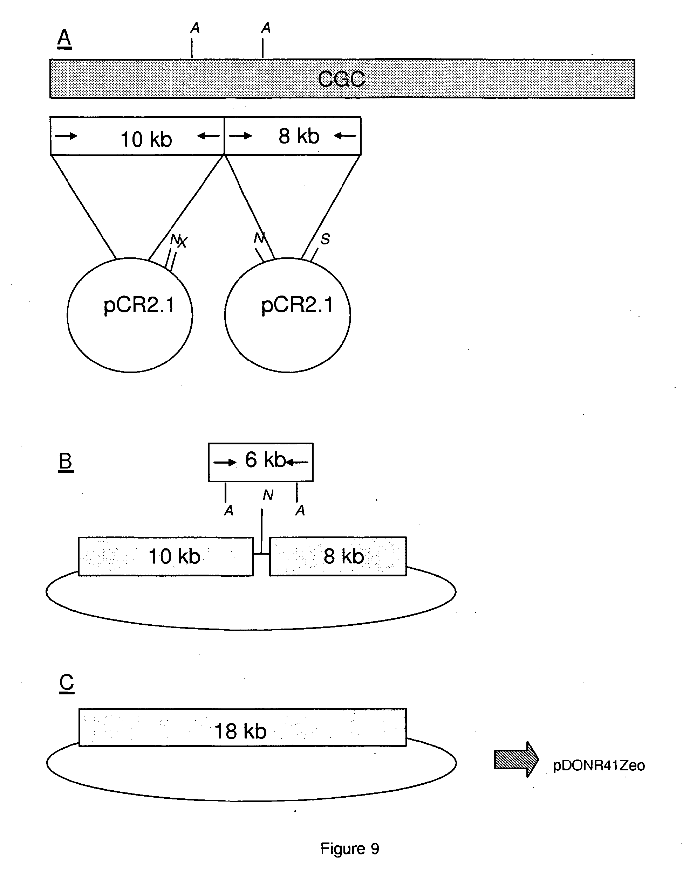

Figure 9 is a schematic overview of cloning strategy for 18 kb left part of the compactin cluster. Panel A: PCR amplification of 10 kb and 8 kb fragments cloned in pCR2.1 TOPO T/A. Panel B: Fusion-cloning of 10 and 8 kb fragments. Notl-Spel digestion of 8 kb fragment, ligated in *Not*l*-Xba*l digested 10 kb plasmid. PCR amplification of internal 6 kb fragment to restore *mlcA* open reading frame. Panel C: Final 18 kb fragment. Transferred via Gateway reaction to pDONR41Zeo. Legend: CGC = Compactin Gene Cluster; N = *Not*l; A = *Acc65*l; X = *Xba*I; S = *Spe*l.

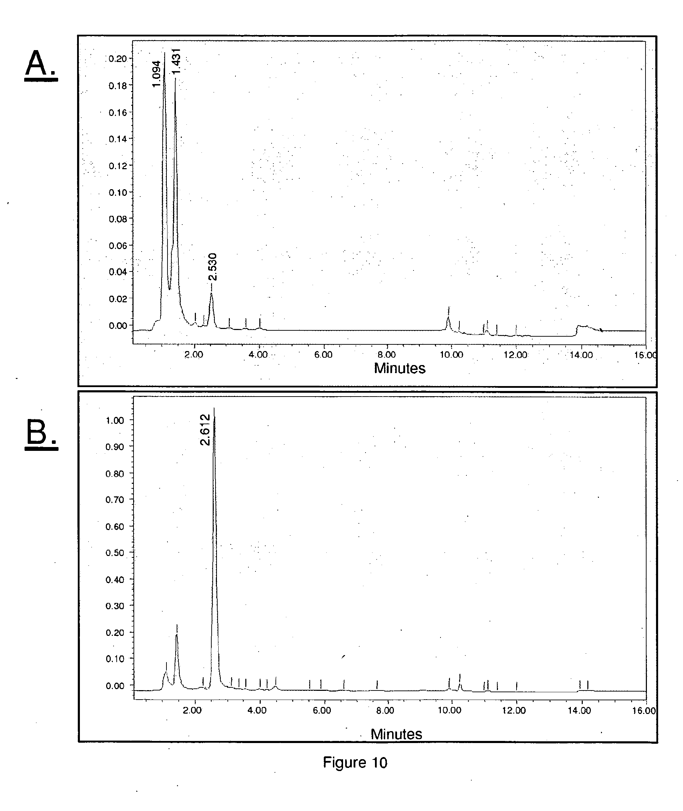

Figure 10 depicts HPLC analyses of the supernatant of fermentation broth. Panel A: *Penicillium chrysogenum* strain deprived of all penicillin biosynthetic gene clusters. Panel B: one of the transformants of the *Penicillium chrysogenum* with integrated compactin gene cluster. A peak corresponding to ML-236A is visible at 2.6 minutes. Legend: X-axis = min; Y-axis = arbitrary units indicating relative peak intensities.

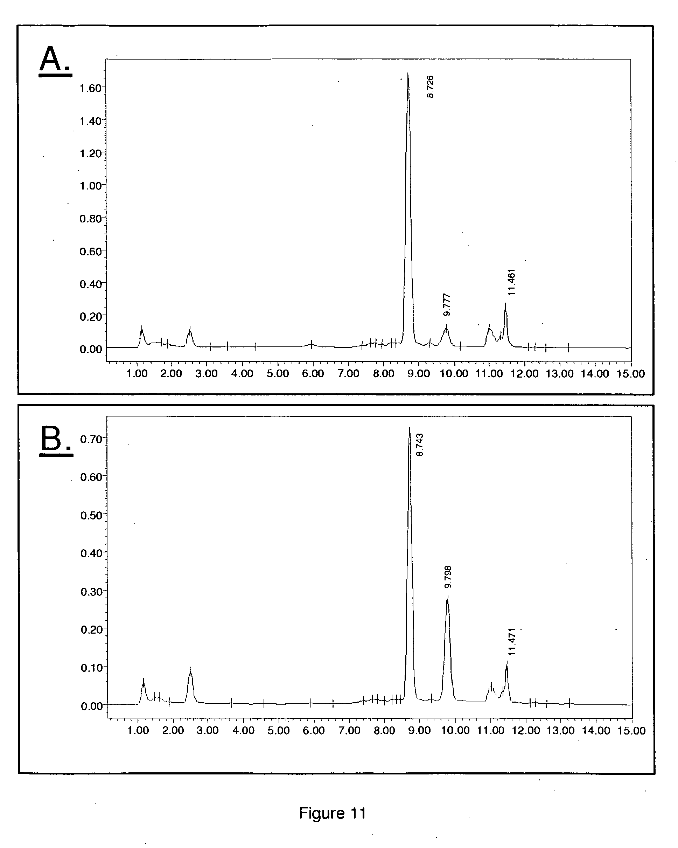

Figure 11 shows the formation of pravastatin as detected by HPLC. Panel A is broth from the *Penicillium chrysogenum* strain equipped with the compactin biosynthetic genes and P-450sca-2 gene from *Streptomyces carbophilus.* Panel B is as panel A, but spiked with pure pravastatin to indicate the peak corresponding to this compound. A peak corresponding to pravastatin is visible at 9.7 minutes. The other main peaks are from ML-236A (8.7 minutes) and compactin (11.5 minutes). Legend: X-axis = minutes; Y-axis = arbitrary units indicating relative peak intensities

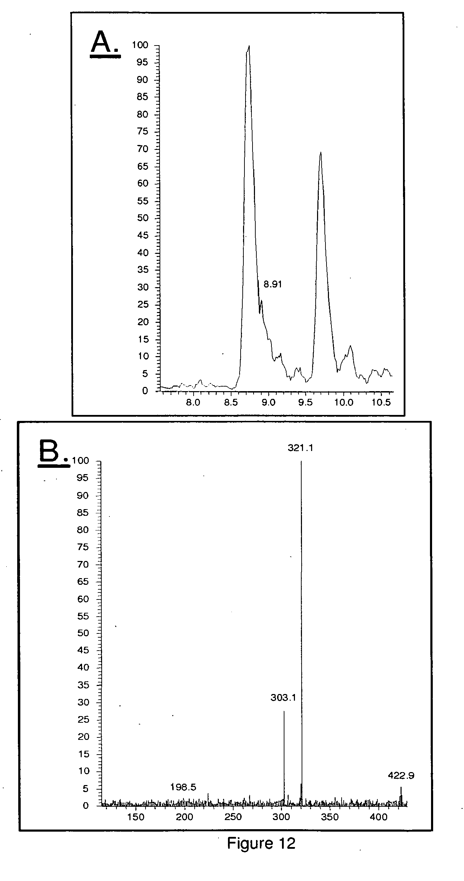

Figure 12 shows the formation of pravastatin as detected by LC/MS/MS. Panel A is broth from the *Penicillium chrysogenum* strain equipped with the compactin biosynthetic genes and P-450sca-2 gene from *Streptomyces carbophilus* analyzed via LC. A peak corresponding to pravastatin is visible at 8.7 minutes. Legend: X-axis = minutes; Y-axis = arbitrary units indicating relative peak intensities. Panel B is the MS/MS analysis of the sample of panel A. The peak pattern corresponds to pravastatin with the correct mass (423) and fragmentation pattern (321, 303 and 198). Legend: X-axis = mass; Y-axis = arbitrary units indicating relative peak intensities.

### EXAMPLES

### General Methods

Standard DNA procedures were carried out as described elsewhere (Sambrook et al., 1989, Molecular cloning: a laboratory manual, 2nd Ed., Cold Spring Harbor Laboratory Press, Cold Spring Harbor, New York). If specific DNA methods were applied these are specified. DNA was amplified using the proofreading enzyme Herculase polymerase (Stratagene). Restriction enzymes were from Invitrogen or New England Biolabs.

### Comparative Example 1

### Statin production by strains described In EP 1,266,967

Manzoni et al. describe the isolation of various statin producing strains in three different publications (1998, Biotechnol Techn 12:529-532; 1999, Biotechnol Lett 21:253-257; EP 1,266,967). While the strains of the former two publications are not available, the strains described in EP 1,266,967 are. *Monascus ruber* GN/33 and *Aspergillus terreus* GN/2218 were obtained from the German National Resource Centre for Biological Material, under collection numbers DSM13554 and DSM13596, respectively. As control strains were used: *Penicillium citrinum* NRRL 8082 and *Aspergillus terreus* ATCC20542. Sporulation of the strains was induced on Potato Dextrose Agar (PDA) slants for 2-7 days at 25°C. The spores were harvested in 0.5% Tween 80 and used to inoculate the pre-culture medium as described in EP 1,266,967 (glucose (25 g/L); casein hydrolysate (10 g/L); yeast extract (15 g/L); NaNO₃ (2 g/L); MgSO₄.7H₂O (0.5 g/L); NaCl (1 g/L); pH 6.2 adjusted with 1M NaOH) and the cultivations were incubated as described in EP 1,266,967. After two days broth from the pre-culture was used to inoculate the main culture. This was done with 10% of the final volume in the medium as described in EP 1,266,967 (glycerol (60 g/L); glucose (20 g/L); peptone (10 g/L); whole soy flour (30 g/L); NaNO₃ (2 g/L); MgSO₄.7H₂O (0.5 g/L); pH 5.8 adjusted with 1 M HCl) and the cultivations were incubated for 144 as described in EP 1,266,967. Broth samples (1 mL) were taken for analysis of the statins produced. To this end CH₃CN (0.5 mL) was added to the sample and the mixture was vortexed vigorously. Cell debris was spun down for 30 min at 14,000 rpm. The supernatant was used for LC/MS analysis. Pravastatin, lovastatin and compactin were used as controls at several dilutions (15, 5, 5, 2.5, 1, 0.5 and 0.25 µg/mL). The LC/MS was performed using the following conditions:

| | | | | | |
|---|---|---|---|---|---|
| Apparatus LC: | Waters Alliance 2795 LC | | | | |
| Mobile phases: | Solvent A: | Water with 0.1 % HCO₂H | | | |
| | Solvent B: | CH₃CN with 0.1% HCO₂H | | | |
| Needle wash: | 50% Milli-Q water + 50% acetonitrile | | | | |
| Gradient: | Time (min) | A% | B% | flow (ml/min) | curve |
| | 0.00 | 85.0 | 15.0 | 1.00 | 1 |
| | 1.50 | 40.0 | 60.0 | 1.00 | 6 |
| | 1.70 | 0.0 | 100.0 | 1.50 | 6 |
| | 1.80 | 85.0 | 15.0 | 1.50 | 6 |
| | 2.00 | 85.0 | 15.0 | 1.00 | 6 |
| | 2.10 | end | | | |
| Column: | Varian, MonoChrom CN, 20 × 2.0 mm, particle size 3 µm | | | | |
| Column temperature: | 25 °C | | | | |
| Injection volume: | 5 µl | | | | |
| Apparatus MS: | Waters ZQ 2000 | | | | |
| Source: | ES+ | | | | |
| Capillary: | 3.50 kV | | | | |
| Cone: | 30 V | | | | |
| Desolvation temp.: | 360 °C | | | | |
| Source temperature: | 140 °C | | | | |
| Extractor: | 2 V | | | | |
| RF lens: | 0.3 V | | | | |
| Cone Gas flow: | 130 l/hour | | | | |
| Desolvation gas flow: | 610 l/hour | | | | |
| LM 1 Resolution: | 15.0 | | | | |
| HM 2 Resolution: | 15.0 | | | | |
| Ion Energy 1: | 0.1 | | | | |
| Multiplier: | 650 V | | | | |
| Scan mass range (m/z) 200 - 600 amu, scan duration 0.20 s, interscan delay 0.05 s | | | | | |
| SIR of 6 channels: 361.3, 413.30, 431.3, 445.3, 447.3, 459.3; dwell 0.07 s; interscan delay 0.05 s | | | | | |

None of the strains produces pravastatin under the conditions as described in EP 1,266,967 (Table 1).

**Table 1 Various statins produced by various strains in mg/L.**

| Strain | | Pravastatin | Compactin | Lovastatin |
|---|---|---|---|---|
| | Retention time (min) | 0.69 | 1.21 | 1.27 |
| | Molecular weight [M+Na]+ | 447.3 | 431.3 | 445.3 |
| *Monascus ruber* DSM13554 | | 0 | 0 | 16.5 |
| *Aspergillus terreus* DSM13596 | | 0 | 0 | 0 |
| *Penicillium citrinum* NRRL 8082 | | 0 | 19.1 | 0 |
| *Aspergillus terreus* ATCC20542 | | 0 | 0 | 40.6 |

### Comparative Example 2

### Statin production by strains described in WO 99/10499

### Isolating the P-450sca-2 gene

Wild type *Penicillium citrinum* cell was equipped with the *Streptomyces carbophilus* P-450sca-2 gene according to WO 99/10499. Thus, strain *Streptomyces carbophilus* FERM-BP 1145 was inoculated in 25 ml liquid medium (in g/L: yeast extract, 1; casamino acids, 4; glycerol, 4; MgSO₄,l; CaCl₂, 0.1; trace elements solution, 2 ml (0.1% ZnSO₄.7H₂O, 0.1% FeSO₄.7H₂O, 0.1% MnCl₂.4H₂O); 0.5% glycine) and incubated at 28°C, 280 rpm, for 72 h. The mycelium was harvested by centrifugation, washed once with 0.7% NaCl and subsequently genomic DNA was isolated according to Hopwood et al. (1985, Genetic manipulation of Streptomyces, a laboratory manual, John Innes Foundation, Norwich). The compactin hydroxylase gene P-450sca-2 was cloned by PCR performed on isolated chromosomal DNA using gene specific forward and reverse primers (SEQ ID NO 1 and 2). The PCR reaction mixture was performed in 50 µl with 125 ng genomic DNA as template, 250 ng of both primers, 2 Units of Pwo polymerase (Boehringer Mannheim), 1x Pwo buffer, 0.2 mM dNTP's and 4 µl DMSO. The blunt PCR DNA-fragment of 1.2 kb was cloned in the vector pCR-blunt (Stratagene) resulting in the constructs pCRP450a. The PCR insert containing the hydroxylase gene in pCRP450a was checked by DNA sequence analysis. After partial digestion with *N*col and complete digestion with *Eco*RV, a 1.2 kb DNA fragment was isolated from pCRP45Oa and cloned in the fungal expression vector pAN8-l (Punt & van den Hondel, 1993, Meth. Enzymology 216:447-457) digested *Nco*l and *Sma*I. The obtained the integration construct pANP450a, checked by restriction analysis, contains the *Streptomyces carbophilus* compactin hydroxylase gene downstream the *Aspergillus nidulans gpdA* promoter. The obtained plasmid is depicted in Figure 2.

### Obtaining Penicillium citrinum transformants with the P-450sca-2 gene

The pANP450a was introduced in *Penicillium citrinum* NRRL 8082 via protoplast transformation, a typical procedure used for filamentous fungi. Details of the method are described as in WO99/10499. Also, we co-introduced pANP450a and pAN7-1 (Punt and Van den Hondel, 1993) and selected for transformants on hygromycin. Hygromycin positive transformants were obtained and re-streaked on fresh hygromycin plates. PCR was applied to verify if hygromycin positive colonies also obtained the pAN450a plasmid. To this end a small piece of colony material was resuspended in 50 µl TE buffer (Sambrook et al., 1989) and incubated at 95°C for 10 min. The cell debris was spun down for 5 min at 3000 rpm. Five µl of the supernatant was used for PCR reaction using the P-450sca-2 specific forward and reverse primers (SEQ ID NO 3 and 4). In total 8 hygromycin resistant, P-450sca-2 positive and stable transformants were obtained.

### Shake flask tests Penicillium citrinum transformants with the P-450sca-2 gene

Sporulation of the strains was induced on Potato Dextrose Agar (PDA) slants for 2-7 days at 25°C. The spores were harvested in 0.5 % Tween80 and used to inoculate the pre-culture medium as in example 1. After two days broth from the pre-culture was used to inoculate the main culture. This was done with 10% of the final volume in the medium as in example 1. Broth samples of 1 ml were taken for analysis of the statins produced. To this end 0.5 ml of acetonitrile was added to the sample and the mixture was vortexed vigorously. Cell debris was spun down for 30 min at 14000 rpm. The supernatant was used for LC/MS analysis as in example 1. As can be seen in Table 2, none of the strains produces pravastatin under the conditions as described in WO 99/10499.

**Table 2 Various statins (in mg/L) produced by Penicillium citrinum WT and transformants with the P-450sca-2 gene from Streptomyces carbophilus.**

| Strain | | Pravastatin | Compactin |
|---|---|---|---|
| | Retention time (min) | 0.69 | 1.21 |
| | Molecular weight [M+Na]+ | 447.3 | 431.3 |
| *Penicillium citrinum* NRRL 8082 | | 0 | 19.1 |
| *Penicillium citrinum* PRA201 | | 0 | 0 |
| *Penicillium citrinum* PRA203 | | 0 | 0 |
| *Penicillium cibinum* PRA204 | | 0 | 0 |
| *Penicillium citrinum* PRA207 | | 0 | 4.5 |

### Example 3

### Isolation of a Penicillium chrysogenum compactin producing host cell

*Penicillium citrinum* is the natural compactin producer (Y. Abe et al., 2002, Mol. Genet. Genomics 267, 636-646). The genes encoding the metabolic pathway are clustered in one fragment on the genome. The functional role of some of these genes is described in literature as well as the fact that over expression of the whole cluster or the specific regulator increases the compactin titer (Abe et al., 2002, Mol. Genet. Genomics 268: 130-137; Abe et al., 2002, Mol. Genet. Genomics, 268:352-361). However, the titers of the wild type strains and the recombinant strains are very low, so a better production host is favorable. As starting strain for a compactin producing host cell any industrial *Penicillium chrysogenum* strain that underwent several rounds of strain improvement to improve the performance of that strain can be used. Examples of these strains are: CBS 455.95 (Gouka et al., 1991, J. Biotechnol. 20:189-200), Panlabs P2 (Lein, 1986, The Panlabs Penicillium strain improvement program, in 'Overprodudion of microbial metabolites', Vanek and Hostalek (eds.), 105-140; Butterworths, Stoneham, MA.), E1 and AS-P-78 (Fierro et al., 1995, Proc. Natl. Acad. Sci. 92:6200-6204), BW1890 and BW1901 (Newbert et al., 1997, J. Ind. Microbiol. 19:18-27). To isolate the β-lactam free derivatives that can be used as compactin producing host cells all copies of genes encoding β-lactam biosynthetic proteins of an industrial *Penicillium chrysogenum* strain must be deleted. As these genes are amplified to multiple copies in the industrial *Penicillium chrysogenum* strain lineages this is not feasible via a single gene deletion. The best approach is to first isolate a derivative of the industrial strain with only one copy of the set of biosynthetic genes. As all the gene amplifications are in direct repeats on the same chromosome (Fierro et al., 1995, Proc. Natl. Acad. Sci. USA 92:6200-6204) there can be recombinations between different repeats resulting in the loss of copies (Newbert et al., 1997, J. Ind. Microbiol. 19:18-27). This is a random process and can be induced via a mutagenic treatment. Derivatives should be screened for reduced penicillin production and reduced copy number of the penicillin biosynthetic genes. If enough derivatives are screened one is able to find these single copy isolates. Those isolates are then used for targeted gene-knockout via homologous recombination.

### Isolation of a single copy isolate

Preparation of *Penicillium chrysogenum* protoplasts was done as described in Cantoral et al., 1987, Biol/Technol. 5:494-497, with glucanex instead of novozyme as lysing enzyme. Protoplasts were separated from the mycelium, washed and plated on mineral medium agar (US 2002/0039758), without phenylacetic acid but supplemented with 15 g/L agar and 1 M saccharose. Regenerating colonies were transferred to plates without saccharose to induce sporulation. Spores were collected, washed with 0.9 mM NaCl, diluted and plated out on YEPD agar plates (10 g/L Yeast Extract, 10 g/L Peptone, 20 g/L glucose and 15 g/L agar). Isolated colonies were transferred to mineral medium agar plates, which served as stock culture plates. 27 random isolates were selected for Southern blot analyses to determine the relative gene-copy number. For this, cells were grown in liquid mineral medium for 48 h at 25°C and 280 rpm. Cells were harvested, washed with 0.9 mM NaCl and the pellet was frozen in liquid N₂. The frozen cells were grinded using a pestle-and-mortar, transferred to a plastic tube and an equal volume of phenol:CHCl₃:isoamylalcohol (25:24:1) was added. This mixture was vortexed vigorously, centrifuged and the aqueous phase was transferred to a fresh tube. This was repeated twice each time using a fresh volume of phenol:CHCl₃:isoamylalcohol (25:24:1). Finally, DNA was isolated from the aqueous phase by ethanol precipitation (Sambrook et al., 1989). DNA (3 µg) was digested with EcoRI, separated on 0.6% agarose and transferred to a nylon membrane by Southern Blotting. As probes *pcbC* and *niaA* were applied. The former is representative for the copy number of penicillin biosynthetic genes and the latter is an internal control (gene encodes for nitrite reductase) that is present as a single copy in *Penicillium chrysogenum* strains. The probe sequences were amplified using gene specific primers (see Table 3) and labeled with the ECL non-radioactive hybridization kit (Amersham) according to the suppliers instructions. The ratio between the intensity of both signals (*pcbC:niaA*) was used to estimate the relative gene copy number of the penicillin gene cluster. The parent strain and the single-copy lab strain Wisconsin 54-1255 were applied as controls.

**Table 3 Primer sequences used to amplify probe sequences**

| **Gene Target** | **Forward primer** | **Reverse primer** |
|---|---|---|
| *PcbC* | SEQ ID NO 5 | SEQ ID NO 6 |
| *NiaA* | SEQ ID NO 7 | SEQ ID NO 8 |

Strains with the lowest ratio were tested for penicillin production in liquid mineral medium with phenoxyacetic acid. Penicillin V production was determined with NMR. All strains with reduced *pcbC:niaA* ratio's also showed reduced penicillin V titers. One strain was selected that underwent a second round of protoplastation, screening and analyses, identical as described above. Again 27 random isolates were selected and analyzed in detail. From the Southern Blot (see Figure 3) several putative 'single copy' penicillin biosynthetic gene cluster candidates (indicated by the arrows) were identified as these showed a comparable *pcbC:niaA* ratio as the lab strain Wisconsin 54-1255. Three of these were selected and tested in shake-flasks for penicillin V production. As controls the original industrial *Penicillium chrysogenum* parent, the intermediate parent, the lab strain Wisconsin 54-1255 and a non-producing derivatives of this lab-strain, npe10 (Cantoral et al., 1993, J. Biol. Chem. 268:737-744) was used. All three isolates showed a drastic reduced penicillin V titer and comparable to the single-copy lab strain Wisconsin 54-1255, therefore it was concluded that these three isolates are industrial single copy isolates, retaining only one copy of the penicillin gene cluster but also all mutations introduced via classical strain improvement (Figure 4).

### Deletion last copy penicillin biosynthetic genes

To inactivate the three biosynthetic genes of the last retained copy of the penicillin gene cluster, the double homologous recombination strategy was applied. Sequences adjacent to the biosynthetic genes were used as flankings to target the *amdS* selection marker to this locus. If double homologous crossover would occur the transformants would use acetamide as sole carbon source (due to the presence of the *amdS* gene), should not produce penicillins and should not hybridize to the pcbC probe. As double homologous crossover is a rare event in *Penicillium chrysogenum* three constructs were produced: one with 3 kb flanks on either side of the *amdS* gene, one with 5 kb and one with 7 kb flanks (see Figure 5). The oligonucleotides applied are listed in Table 4.

**Table 4 Primer sets for constructing double homologous crossover cassettes**

| **Flank** | **Size (kb)** | **Forward primer** | | **Reverse primer** | |
|---|---|---|---|---|---|
| | | SEQ ID NO | Restriction enzyme introduced | SEQ ID NO | Restriction enzymes introduced |
| Left | 7 | 9 | *Acc*65l | 10 | *Not*l |
| Left | 5 | 11 | *Acc*G5l | 10 | *Not*l |
| Left | 3 | 12 | *Acc*65l | 10 | *Not*l |
| Right | 3 | 13 | *Not*l | 14 | *Eco*52l, *Acc*65l |
| Right | 5 | 13 | *Not*l | 15 | *Eco*52l, *Acc*65l |
| Right | 7 | 13 | *Not*l | 16 | *Eco*52l, *Acc*65l |

Following PCR amplification the fragments were cloned in pCRXL via TOPO T/A cloning (Invitrogen). Subsequently all three left flankings (3, 5 and 7 kb) were digested with *Acc*651 and *Not*l followed by ligation in pBluescript II SK+ (Invitrogen) pre-digested Acc651 and *Not*l. The obtained left-flanking plasmids were digested with Notl to facilitate cloning of the right flanks, which were pre-digested with Notl and *Eco*521. The obtained 3, 5 and 7 kb flanking-plasmids all had a unique Notl site between the left and right flanks, which was used to clone the *amdS* gene as selection marker. This was obtained by digesting pHELY-A1 (described in WO 04/106347) with *Notl* and isolating the 3.1 kb *PgpdA-AnamdS* expression cassette. The thus obtained deletion fragments were isolated following digestion with *Kpn*l and transformed to the penicillin gene cluster single copy isolates. Transformants were selected on their ability to grow on acetamide selection plates and afterwards screened for antibiotic production by replica plating the colonies on mineral medium and overlaying them after 4 days of growth with a β-lactam sensitive indicator organism, *Escherichia coli* strain ESS. If colonies still produced β-lactams this inhibits the growth of the *Escherichia coli.* 22 out of the 27.076 transformants tested gave no inhibition zone (0.08%) and were selected for further analyses. These 22 isolates were analyzed via colony PCR with three primer sets: *niaA,* as an internal control for a single copy gene; *amdS,* for the selection marker; *penDE,* as indicator for the presence or absence of the penicillin biosynthetic genes.

**Table 5 Primer sequences used to for colony PCR**

| **Gene** | **FWD primer** | **REV primer** | **Fragment size (bp)** |
|---|---|---|---|
| *NiaA* | SEQ ID NO 7 | SEQ ID NO 8 | 251 |
| *AmdS* | SEQ ID NO 17 | SEQ ID NO 18 | 653 |
| *penDE* | SEQ ID NO 19 | SEQ ID NO 20 | 1000 |

**Table 6 Colony PCR on putative Penicillium chrysogenum strain isolates**

| **Strain** | **Fragment used for deletion with** | ***niaA*** | ***amdS*** | ***penDE*** |
|---|---|---|---|---|
| Npe10 | - | + | - | - |
| Wisconsin54-1255 | - | + | - | + |
| CBS 455.95 | - | + | - | + |
| Deletion mutant | 3 kb flanks | + | + | - |
| Deletion mutant | 3 kb flanks | + | + | - |
| Deletion mutant | 5 kb flanks | + | + | - |
| Deletion mutant | 5 kb flanks | + | + | - |
| Deletion mutant | 5 kb flanks | + | + | - |
| Deletion mutant | 5 kb flanks | + | + | - |
| Deletion mutant | 7 kb flanks | + | + | - |
| Deletion mutant | 7 kb flanks | + | + | - |
| Deletion mutant | 7 kb flanks | + | + | - |
| Deletion mutant | 7 kb flanks | + | - | - |
| Deletion mutant | 3 kb flanks | + | + | - |
| Deletion mutant | 3 kb flanks | + | + | - |
| Deletion mutant | 3 kb flanks | + | + | - |
| Deletion mutant | 5 kb flanks | + | + | - |
| Deletion mutant | 5 kb flanks | + | + | - |
| Deletion mutant | 7 kb flanks | + | + | - |
| Deletion mutant | 7 kb flanks | + | + | - |
| Deletion mutant | 7 kb flanks | + | + | - |
| Deletion mutant | 7 kb flanks | + | + | - |
| Deletion mutant | 7 kb flanks | + | + | - |
| Deletion mutant | 7 kb flanks | + | - | - |
| Deletion mutant | 7 kb flanks | + | + | - |

All 22 putative mutants gave no signal for the gene *penDE,* encoding acyltransferase, catalyzing the last step in the penicillin biosynthesis. Two mutants gave no signal for amdS, suggesting spontaneous loss of the selection marker gene. It was concluded that all 22 isolates are derivatives of industrial *Penicillium chrysogenum* strains without β-lactam biosynthetic genes and qualify as possible host for compactin production.

### Shake Flask tests

All 22 mutants were tested in shake flasks to confirm the penicillin-negative phenotype by inoculating the mutants in liquid mineral medium with phenyl acetic acid as precursor. Samples were analyzed with NMR confirming that none of the mutants was capable of producing penicillin G (Figure 6) and therefore it was concluded that all were correct *Penicillium chrysogenum* β-lactam free isolates.

### Isolation of compactin gene set

Chromosomal DNA was isolated from *Penicillium citrinum* NRRL8082. As the full gene cluster is difficult to amplify via PCR due to its size (38 kb; Figure 7), it was divided in three fragments: 18 kb, 14 kb and 6 kb. The 14 and 6 kb fragments, were readily PCR amplified (Figure 8) and cloned using Gateway (Invitrogen) into the entry vectors pDONRP4-P1R and pDONR221 with a so-called LR gateway reaction according to the suppliers' instructions. The 18 kb fragment was cloned in a two-step procedure. First, a 10 and an 8 kb fragment were amplified. Both fragments were cloned separately in pCR2.1 TOPO T/A (Invitrogen) and subsequently fused together via restriction enzyme cloning and ligation (see Figure 9). Finally, the fragment was transferred to the pDONR41Zeo vector using Gateway technology. The amplified fragments were verified via sequencing. Using a so-called Multi-site Gateway Reaction (see manual Invitrogen) these three gene fragments containing all the genes of the compactin biosynthetic gene clusters can be recombined into one fragment, spanning the whole region.

**Table 7 Oligonucleotides used to amplify the compactin biosynthetic gene cluster**

| **Target DNA** | **Forward primer** | | **Reverse primer** | |
|---|---|---|---|---|
| | Cluster sequence | Gateway Sequence | Cluster sequence | Gateway Sequence |
| Left part of the compactin cluster (10 kb fragment) | SEQ ID 21 | attB4 | SEQ ID 22 | - |
| Left part of the compactin cluster (8 kb fragment) | SEQ ID 23 | - | SEQ ID 24 | attB1 |
| Internal 6 kb of left part of compactin cluster | SEQ ID 25 | - | SEQ ID 26 | - |
| Middle part of the compactin cluster (14 kb fragment) | SEQ ID 27 | attB1 | SEQ ID 28 | attB2 |
| Right part of the compactin cluster (6 kb fragment) | SEQ ID 29 | attB2 | SEQ ID 30 | attB3 |

### Penicillium transformation

The three compactin gene cluster fragments were co-transformed to the *Penicillium chrysogenum* platform strain with a *ble* expression cassette encoding for a protein that mediates phleomycin resistance. This cassette can be isolated as a 1.4 kb *Sal*l fragment from pAMPF7 (Fierro et al., 1996, Curr. Genet. 29:482-489). Selection of transformants was done on mineral medium agar plates with 50 µg/mL Phleomycin and 1 M saccharose. Phleomycin resistant colonies appearing on these protoplast regeneration plates were re-streaked on fresh phleomycin agar plates without the saccharose and grown until sporulation. The phleomycin resistant transformants were screened via colony PCR for the presence of one or more compactin gene fragments. For this, a small piece of colony material was suspended in 50 µl TE buffer (Sambrook et al., 1989) and incubated for 10 minutes at 95 C. To discard the cell debris the mixture was centrifuged for 5 minutes at 3000 rpm. The supernatant (5 µl) was used as a template for the PCR-reaction with SUPER TAQ from HT Biotechnology Ltd. The PCR-reactions were analyzed on the E-gel96 system from Invitrogen.

**Table 8 Oligonucleotides used in colony PCR for determining the presence of the compactin biosynthetic gene cluster**

| **Target DNA** | **Forward primer** | **Reverse primer** |
|---|---|---|
| 18 kb fragment | SEQ ID NO 31 | SEQ ID NO 32 |
| 14 kb fragment | SEQ ID NO 33 | SEQ ID NO 34 |
| 6 kb fragment | SEQ ID NO 35 | SEQ ID NO 36 |
| *niaA* | SEQ ID NO 7 | SEQ ID NO 8 |

First, the presence of the 18 kb fragment was checked. Out of 480 colonies checked 112 had the 18 kb fragment stably integrated (∼23%). Subsequently, the presence of the other two fragments (14 and 6 kb) was verified. Forty-five of the 18 kb-positive transformants also had both other parts of the compactin gene cluster and thereby qualified as putative compactin production strains.

### Compactin shake Flask tests

The *Penicillium chrysogenum* platform strain transformants with the full compactin gene cluster were evaluated in liquid mineral media (without phenylacetic acid) for the presence of (hydrolyzed) compactin and ML-236A (6-(2-(1,2,6,7,8,8a-hexahydro-8-hydroxy-2-methyl-1-naphthalenyl)ethyl)tetrahydro-4-hydroxy-2H-pyran-2-one). After 168 h of cultivation at 25°C in 25 ml the supernatant was analyzed with HPLC using the following equipment and conditions:

| | |
|---|---|
| Column: | Waters XTerra RP18 |
| Column Temp: | Room temperature |
| Flow: | 1 ml/min |
| Injection volume: | 10 µl |
| Tray temp: | Room temperature |
| Instrument: | Waters Alliance 2695 |
| Detector: | Waters 996 Photo Diode Array |
| Wavelength: | 238 nm |
| Eluens: | A: 33% CH₃CN, 0.025% CF₃CO₂H in milliQ water |
| | B: 80% CH₃CN in milliQ water |
| | C: milliQ water |

Two different gradients were used:

| **Gradient 1** | | | |
|---|---|---|---|
| **Time (min)** | **Eluens (%)** | | |
| | **A** | **B** | **C** |
| | | | |
| 0.0-8.0 | 100 | 0 | 0 |
| 8.0-8.1 | 100→0 | 0→100 | 0 |
| 8.1 -12 | 0 | 100 | 0 |
| 12.0 -13.0 | 0→100 | 100→0 | 0 |
| 13.0-14.0 | 100 | 0 | 0 |

| **Gradient 2** | | | |
|---|---|---|---|
| **Time (min)** | **Eluens (%)** | | |
| | **A** | **B** | **C** |
| | | | |
| 0.0 - 5.0 | 50 | 0 | 50 |
| 5.0 - 5.1 | 50→100 | 0 | 50→0 |
| 5.1-9.0 | 100 | 0 | 0 |
| 9.0 - 9.1 | 100→0 | 0→100 | 0 |
| 9.1 -13.0 | 0 | 100 | 0 |
| 13.0 -13.1 | 0→50 | 100→0 | 0→50 |
| 13.1-15.0 | 50 | 0 | 50 |

| | | |
|---|---|---|
| Retention times (gradient 1): | Hydrolyzed compactin | 10.4 min |
| | Compactin | 10.9 min |
| | ML-236A | 2.6 min |
| Retention times (gradient 2): | Hydrolyzed compactin | 11.5 min |
| | Compactin | 11.8 min |
| | ML-236A | 8.6 min |

The wild type *Penicillium citrinum* strains barely produce any statins, while the *Penicillium chrysogenum* transformants produce significant amounts (Table 9). An example of the HPLC chromatograms is shown in Figure 10. These data confirm the high potential of using derivatives of *Penicillium chrysogenum* industrial production strains as host cells for the production compactin.

**Table 9 Statin levels (compactin and ML-236A) produced by different strains.**

| **Strain** | **Compactin (mg/L)** | **ML-236A (mg/L)** |
|---|---|---|
| *Penicillium citrinum* NRRL8082 | <0,5 | 0 |
| *Penicillium citrinum* NRRL8082 | <0,5 | 0 |
| *Penicillium citrinum* NRRL8082 | 0.9 | 0 |
| *Penicillium citrinum* NRRL8082 | <0,5 | 0 |
| *Penicillium chrysogenum* β-lactam free isolates | 0 | 0 |
| *Penicillium chrysogenum* β-lactam free isolates | 0 | 0 |
| Compactin cluster transformant | 10 | 465 |
| Compactin cluster transformant | 7 | 420 |

### Example 4

### Isolation of a Penicillium chrysogenum pravastatin producing host cell

### Strain construction

The plasmid pAN450a as described in example 2 was used to transform the *Penicillium chrysogenum* β-lactam free strains as described in Example 3. To this end all three compactin gene fragments, pAN450a and the phleomycin gene cassette were co-transformed to *Penicillium chrysogenum* host cells as described in Example 3. After initial selection on phleomycin selection plates, colonies were re-streaked on selective plates and used for colony PCR. First, the presence of the compactin genes was confirmed, as described in Example 3 using the primers as depicted in Table 8. Second, the presence of the P-450sca-2 gene was confirmed, described in Example 2 using the primers of SEQ ID NO 3 and 4. Two transformants, T1.48 and T1.37, were shown to contain all genes and were subsequently used for shake flask analysis.

### Pravastatin shake flask cultivations and HPLC analysis

Both transformants were cultivated in fungal mineral urea-free medium containing (g/L): glucose (5); lactose (80); (NH₄)₂SO₄ (2.5); Na₂SO₄ (2.9); KH₂PO₄ (5.2); K₂HPO₄ (4.8) and 10 ml/l of a solution containing citric acid (150); FeSO₄.7H₂O (15); MgSO₄.7H₂O (150); H₃BO₃, 0.0075; CuSO₄.5H₂O, 0.24; CₒSO₄.7H₂O, 0.375; ZnSO₄.7H₂O (0.5); MnSO₄.H₂O (2.28) and CaCl₂.2H₂O (0.99); pH before sterilization 6.5. The cultures are incubated at 25°C in an orbital shaker at 280 rpm for 120 h. HPLC analysis was the same as in Example 3. Using gradient 2 the retention times were: hydrolyzed compactin 11.5 min; pravastatin 9.7 min and ML-236A 8.6 min. While the parent *Penicillium chrysogenum* strains, the *Penicillium chrysogenum* compactin transformants, the wild type *Penicillium citrinum* strains, nor the *Penicillium citrinum* transformants of example 2 produce any pravastatin, the *Penicillium chrysogenum* transformants equipped with both the compactin and P-450sca-2 genes do produce pravastatin. An example of the HPLC chromatograms of transformant T1.47 is shown in Figure 11

### LC/MS/MS analysis of transformant T1.48 broth

To verify that the peak identified via HPLC was indeed pravastatin, LC/MS/MS analysis was performed. To this end the samples are diluted 1:1 with water and injected on the LC/MS/MS system as follows:

| | | | | | |
|---|---|---|---|---|---|
| LC | Eluens: | A: 0.1% HCO₂H in water; B: 0.1% HCO₂H in CH₃CN | | | |
| | Gradient: | T (min) | Flow (ml/min) | A(%) | B (%) |
| | | 0.0 | 0.2 | 70 | 30 |
| | | 20.0 | 0.2 | 10 | 90 |
| | | 20.1 | 0.2 | 70 | 30 |
| | | 25.0 | 0.2 | 70 | 30 |
| | Column : | Varian Inertsil 3 ODS 3 CP22568 150*2.1 mm 3µm | | | |
| | Column temp.: | 55°C | | | |
| | Injection volume: | 50 µl | | | |
| | Tray temp.: | 4°C | | | |
| MS | Instrument: | LCQ (SM05) | | | |
| | LC/MS: | ESI/neg for pravastatin | | | |
| | LC/MS/MS: | Pravastatin iw = 2.1 aa = 25% | | | |
| | Retention time: | Pravastatin 8.7 min | | | |
| | m/z range: | 200-800 | | | |
| | Micro scans: | 1 | | | |
| | Inject time: | 500 ms | | | |

Pravastatin elutes at 8.7 minutes using the LC method. Pravastatin is characterized in ESI/neg mode by the deprotonated molecule [M-H]- at m/z 423. In the MS/MS mode characteristic loss of 102 (methylbutyric acid) and 120 (102-H₂O) is observed (Figure 12), confirming that this *Penicillium chrysogenum* transformant equipped with the compactin and P-450sca-2 genes is capable of producing pravastatin in a single fermentation cultivation starting from simple and defined nutrients.

## Claims

1. Method for producing pravastatin comprising the steps of:
(a) transforming a *Penicillium chrysogenum* cell with a polynucleotide comprising genes mlcA, mlcB, mlcC, mlcD, mlcE, mlcF, mlcG and mlcH;
(b) transforming said *Penicillium chrysogenum* cell with a polynucleotide comprising a gene encoding compactin hydroxylase;
(c) selecting clones of transformed cells, and;
(d) cultivating said selected cells.

2. Method according to claim 1 further comprising the feeding of nutrient sources to said cultivated cells.

3. Method according to any one of claims 1 to 2 further comprising isolating said pravastatin from said cultivated cells.

4. Method according to any one of claims 1 to 3 wherein said *Penicillium chrysogenum* cell is transformed with a polynucleotide comprising a DNA sequence affecting the expression of the genes of step (a) and/or step (b).

5. Method according to anyone of claims 1 to 4 wherein said genes encoding the minimal requirements for compactin biosynthesis are not mlcR and wherein the original *Penicillium chrysogenum* promoters are replaced by promoters which are not under control by the pathway specific transcription regulator, encoded by the gene *mlcR.*

6. Method according to anyone of claims 1 to 5 wherein said hydroxylase gene is a P-450 gene or a homologue thereof.

7. Method according to claim 6 wherein said P-450 gene is P-450sca-2 from *Streptomyces carbophilus* or a homologue thereof.

8. Method according to any one of claims 1 to 7 further comprising a ferredoxin gene.

9. Method according to any one of claims 1 to 8 wherein said *Penicillium chrysogenum* cell comprises homologous proteins with improved kinetic features.

10. Method according to anyone of claims 1 to 9 wherein said *Penicillium chrysogenum* cell comprises a redox regenerating system.

## Patentansprüche

1. Verfahren zur Herstellung von Pravastatin, umfassend die folgenden Schritte:
(a) Transformieren einer *Penicillium-chrysogenum-*Zelle mit einem Polynukleotid umfassend die Gene micA, micB, micC, micD, micE, micF, micG und micH;
(b) Transformieren der *Penicillium-chysogenum*-Zelle mit einem Polynukleotid, das ein Gen umfasst, das für die Compactinhydroxylase codiert;
(c) Selektieren von Klonen von transformierten Zellen und
(d) Kultivieren der selektierten Zellen.

2. Verfahren nach Anspruch 1, das weiterhin das Bereitstellen von Nährstoffquellen für die kultivierten Zellen umfasst.

3. Verfahren nach einem der Ansprüche 1 bis 2, das weiterhin das Isolieren des Pravastatins aus den kultivierten Zellen umfasst.

4. Verfahren nach einem der Ansprüche 1 bis 3, wobei die *Penicillium-chrysogenum-*Zelle mit einem Polynukleotid, das eine DNA-Sequenz, die die Expression der Gene von Schritt (a) und/oder Schritt (b) beeinflusst, umfasst, transformiert wird.

5. Verfahren nach einem der Ansprüche 1 bis 4, wobei es sich bei den Genen, die für die Minimalerfordernisse für die Compactinbiosynthese codieren, nicht um micR handelt und wobei die ursprünglichen *Penicillium-*chrysogenum-Promoter durch Promoter ersetzt werden, die nicht unter der Kontrolle des von dem Gen micR codierten biosynthesewegspezifischen Transkriptionsregulators stehen.

6. Verfahren nach einem der Ansprüche 1 bis 5, wobei es sich bei dem Hydroxylasegen um ein P-450-Gen oder ein Homolog davon handelt.

7. Verfahren nach Anspruch 6, wobei es sich bei dem P-450-Gen um P-450sca-2 aus *Streptomyces carbophilus* oder ein Homolog davon handelt.

8. Verfahren nach einem der Ansprüche 1 bis 7, das weiterhin ein Ferredoxin-Gen umfasst.

9. Verfahren nach einem der Ansprüche 1 bis 8, wobei die *Penicillium-chrysogenum-*Zelle homologe Proteine mit verbesserten Kinetikmerkmalen umfasst.

10. Verfahren nach einem der Ansprüche 1 bis 9, wobei die *Penicillium-chrysogenum-*Zelle ein Redoxregenerationssystem umfasst.

## Revendications

1. Procédé pour produire de la pravastatine comprenant les étapes consistant à :
(a) transformer une cellule de *Penicillium chrysogenum* avec un polynucléotide comprenant les gènes mIcA, mlcB, mlcC, mlcD, mlcE, mlcF, mlcG et mlcH ;
(b) transformer ladite cellule de *Penicillium chrysogenum* avec un polynucléotide comprenant un gène codant pour la compactine hydroxylase ;
(c) sélectionner des clones de cellules transformées, et ;
(d) cultiver lesdites cellules sélectionnées.

2. Procédé selon la revendication 1 comprenant en outre l'alimentation de sources de nutriment auxdites cellules cultivées.

3. Procédé selon l'une quelconque des revendications 1 à 2 comprenant en outre l'isolement de ladite pravastatine à partir desdites cellules cultivées.

4. Procédé selon l'une quelconque des revendications 1 à 3 dans lequel ladite cellule de *Penicillium chrysogenum* est transformée avec un polynucléotide comprenant une séquence d'ADN affectant l'expression des gènes de l'étape (a) et/ou l'étape (b).

5. Procédé selon l'une quelconque des revendications 1 à 4 dans lequel lesdits gènes codant pour les exigences minimales pour la biosynthèse de compactine ne sont pas mlcR et dans lequel les promoteurs originaux de *Penicillium chrysogenum* sont remplacés par des promoteurs qui ne sont pas sous le contrôle du régulateur de transcription spécifique de la voie, codé par le gène *mlcR.*

6. Procédé selon l'une quelconque des revendications 1 à 5 dans lequel ledit gène d'hydroxylase est un gène P-450 ou un homologue de ceux-ci.

7. Procédé selon la revendication 6 dans lequel ledit gène de P-450 est P-450sca-2 de *Streptomyces carbophilus* ou un homologue de celui-ci.

8. Procédé selon l'une quelconque des revendications 1 à 7 comprenant en outre un gène de ferredoxine.

9. Procédé selon l'une quelconque des revendications 1 à 8 dans lequel ladite cellule de *Penicillium chrysogenum* comprend des protéines homologues ayant des caractéristiques cinétiques améliorées.

10. Procédé selon l'une quelconque des revendications 1 à 9 dans lequel ladite cellule de *Penicillium chrysogenum* comprend un système de régénération redox.


### SEQUENCE LISTING

<110> DSM IP Assets B.V.
<120> PRODUCTION OF COMPOUNDS IN A RECOMBINANT HOST
<130> 25630WO
<160> 36
<170> PatentIn version 3.1
<210> 1
   <211> 28
   <212> DNA
   <213> Artificial sequence
<220>
   <223> Description of artificial sequence: synthetic primer
<400> 1
   CACCATGGCC GAGATGACAG AGAAAGCC 28
<210> 2
   <211> 28
   <212> DNA
   <213> Artificial sequence
<220>
   <223> Description of artificial sequence: synthetic primer
<400> 2
   CAGGATCCCG CTCGGTCACC AGGTGACC 28
<210> 3
   <211> 22
   <212> DNA
   <213> Artificial sequence
<220>
   <223> Description of artificial sequence: synthetic primer
<400> 3
   ATCACCAAGC TGGAGTCCGA AC 22
<210> 4
   <211> 22
   <212> DNA
   <213> Artificial sequence
<220>
   <223> Description of artificial sequence: synthetic primer
<400> 4
   GGATGAGCTG TCCGTCGATC TC 22
<210> 5
   <211> 21
   <212> DNA
   <213> Artificial sequence
<220>
   <223> Description of artificial sequence: synthetic primer
<400> 5
   GATTGGCGCT CCTCGTTCAC C 21
<210> 6
   <211> 50
   <212> DNA
   <213> Artificial sequence
<220>
   <223> Description of artificial sequence: synthetic primer
<400> 6
   CCATTATTTT TCTAGTCGAC ATGGCATCGA TTCCCAAGGC CAATGTCCCC 50
<210> 7
   <211> 25
   <212> DNA
   <213> Artificial sequence
<220>
   <223> Description of artificial sequence: synthetic primer
<400> 7
   CACAGAGAAT GTGCCGTTTC TTTGG 25
<210> 8
   <211> 24
   <212> DNA
   <213> Artificial sequence
<220>
   <223> Description of artificial sequence: synthetic primer
<400> 8
   TCACATATCC CCTACTCCCG AGCC 24
<210> 9
   <211> 45
   <212> DNA
   <213> Artificial sequence
<220>
   <223> Description of artificial sequence: synthetic primer
<400> 9
   GTTACACGCT TTGATTCTGT GGGTACCGAT GTTATATTCA GCTAC 45
<210> 10
   <211> 44
   <212> DNA
   <213> Artificial sequence
<220>
   <223> Description of artificial sequence: synthetic primer
<400> 10
   CCCAATAGCG GCCGCAGTTG ATAATATCAA TATCTAAAAC TCCC 44
<210> 11
   <211> 42
<212> DNA
   <213> Artificial sequence
<220>
   <223> Description of artificial sequence: synthetic primer
<400> 11
   GGCATATACG AGCATGGTAC CAGGGACAGA TGCCCATCCT TG 42
<210> 12
   <211> 40
   <212> DNA
   <213> Artificial sequence
<220>
   <223> Description of artificial sequence: synthetic primer
<400> 12
   GTATAAAAGG GGAGGGTACC GGGAAAGATT TGTGGGCCTG 40
<210> 13
   <211> 45
   <212> DNA
   <213> Artificial sequence
<220>
   <223> Description of artificial sequence: synthetic primer
<400> 13
   GTATGTAGCT GCGGCCGCCT CCGTCTTCAC TTCTTCGCCC GCACT 45
<210> 14
   <211> 50
   <212> DNA
   <213> Artificial sequence
<220>
   <223> Description of artificial sequence: synthetic primer
<400> 14
   CCGCCTTCCT CACTAACCGG CCGGCAGGTA CCGATGGACT CAGCATTATC 50
<210> 15
   <211> 47
   <212> DNA
   <213> Artificial sequence
<220>
   <223> Description of artificial sequence: synthetic primer
<400> 15
   CTCTAGAATG CTACGGCCGT TCGAGGTACC TTATAGGAAA AAGGTAG 47
<210> 16
   <211> 45
   <212> DNA
   <213> Artificial sequence
<220>
   <223> Description of artificial sequence: synthetic primer
<400> 16
   CCTTTTCGCT GAGCGGCCGC AATCACAGGT ACCGTTTTTG TCGTC 45
<210> 17
   <211> 24
   <212> DNA
   <213> Artificial sequence
<220>
   <223> Description of artificial sequence: synthetic primer
<400> 17
   ATGCCTCAAT CCTGGGAAGA ACTG 24
<210> 18
   <211> 24
   <212> DNA
   <213> Artificial sequence
<220>
   <223> Description of artificial sequence: synthetic primer
<400> 18
   CTTGACGTAG AAGACGGCAC CGGC 24
<210> 19
   <211> 36
   <212> DNA
   <213> Artificial sequence
<220>
   <223> Description of artificial sequence: synthetic primer
<400> 19
   CCCGCAGCAC ATATGCTTCA CATCCTCTGT CAAGGC 36
<210> 20
   <211> 21
   <212> DNA
   <213> Artificial sequence
<220>
   <223> Description of artificial sequence: synthetic primer
<400> 20
   ATGACAAACA TCTCATCAGG G 21
<210> 21
   <211> 53
   <212> DNA
   <213> Artificial sequence
<220>
   <223> Description of artificial sequence: synthetic primer
<400> 21
   GGGGACAACT TTGTATAGAA AAGTTGAAGG ATGACTATTC CAGTGATTAG CAC 53
<210> 22
   <211> 27
   <212> DNA
   <213> Artificial sequence
<220>
   <223> Description of artificial sequence: synthetic primer
<400> 22
   GAGAAGACGA AACTCGTGCT TTGAGTG 27
<210> 23
   <211> 27
   <212> DNA
   <213> Artificial sequence
<220>
   <223> Description of artificial sequence: synthetic primer
<400> 23
   CACTCAAAGC ACGAGTTTCG TCTTCTC 27
<210> 24
   <211> 53
   <212> DNA
   <213> Artificial sequence
<220>
   <223> Description of artificial sequence: synthetic primer
<400> 24
   GGGGACTGCT TTTTTGTACA AACTTGAAGG GAGTACTTGT GTCCACGTCG TTG 53
<210> 25
   <211> 22
   <212> DNA
   <213> Artificial sequence
<220>
   <223> Description of artificial sequence: synthetic primer
<400> 25
   GTGGTAGGCG GCCAGGTAGA AC 22
<210> 26
   <211> 22
   <212> DNA
   <213> Artificial sequence
<220>
   <223> Description of artificial sequence: synthetic primer
<400> 26
   CAGCATCTTC GTGGAGGTGC GC 22
<210> 27
   <211> 59
   <212> DNA
   <213> Artificial sequence
<220>
   <223> Description of artificial sequence: synthetic primer
<400> 27
   GGGGACAAGT TTGTACAAAA AAGCAGGCTA ACCCGCCTTC CGACTACATA TCCACAATC 59
<210> 28
   <211> 56
   <212> DNA
   <213> Artificial sequence
<220>
   <223> Description of artificial sequence: synthetic primer
<400> 28
   GGGGACCACT TTGTACAAGA AAGCTGGGTA CTCAGGAATG AATCAGATCA ACATTC 56
<210> 29
   <211> 54
   <212> DNA
   <213> Artificial sequence
<220>
   <223> Description of artificial sequence: synthetic primer
<400> 29
   GGGGACAGCT TTCTTGTACA AAGTGGAAGT ATCAGGATTG ATGCCTGAAA CATC 54
<210> 30
   <211> 53
   <212> DNA
   <213> Artificial sequence
<220>
   <223> Description of artificial sequence: synthetic primer
<400> 30
   GGGGACAACT TTGTATAATA AAGTTGAGAT CTGCTGGTAG ACTAGAGCCT GCC 53
<210> 31
   <211> 27
   <212> DNA
   <213> Artificial sequence
<220>
   <223> Description of artificial sequence: synthetic primer
<400> 31
   CACAGGAATC ACAGCAGAAC AGTCATC 27
<210> 32
   <211> 25
   <212> DNA
   <213> Artificial sequence
<220>
   <223> Description of artificial sequence: synthetic primer
<400> 32
   TCCCATTTGC TGTTGATGGA GCAGC 25
<210> 33
   <211> 31
   <212> DNA
   <213> Artificial sequence
<220>
   <223> Description of artificial sequence: synthetic primer
<400> 33
   GATCTGAGAT GTCACATGCG TGTAGATAGA C 31
<210> 34
   <211> 27
   <212> DNA
   <213> Artificial sequence
<220>
   <223> Description of artificial sequence: synthetic primer
<400> 34
   CAATTGATCT TCTCTCGTGG CAAAGAG 27
<210> 35
   <211> 22
   <212> DNA
   <213> Artificial sequence
<220>
   <223> Description of artificial sequence: synthetic primer
<400> 35
   TGGTTGCGAA GGCTGCAAAG AC 22
<210> 36
   <211> 26
   <212> DNA
   <213> Artificial sequence
<220>
   <223> Description of artificial sequence: synthetic primer
<400> 36
   TGTACACGCT GACCTCGCAT ATGAAG 26